# EUROPEAN PATENT APPLICATION

(11) **EP 4 616 866 A1**
(43) Date of publication of application: **17.09.2025**
(21) Application number: 23888692.3
(22) Date of filing: 07.11.2023
(51) Int. Cl.: A61K 45/00, A61K 39/395, A61P 21/00, A61P 21/02, A61P 25/00, A61P 25/02, A61P 25/20, A61P 25/28, A61P 43/00, C07K 16/18, C12N 15/13

(54) **AGENT FOR PREVENTING OR TREATING DISEASE ASSOCIATED WITH ACCUMULATION OF ABNORMAL PROTEIN AGGREGATES**

(30) Priority: 07.11.2022 JP 2022178369; 21.07.2023 JP 2023119403
(71) Applicant: The University of Osaka, Suita-shi, Osaka 565-0871 (JP); Mitsubishi Tanabe Pharma Corporation, Osaka-shi, Osaka 541-8505 (JP)
(72) Inventor: OKUNO, Tatsusada, Suita-shi, Osaka 565-0871 (JP); SHIMIZU, Mikito, Suita-shi, Osaka 565-0871 (JP); YAMASHITA, Toshihide, Suita-shi, Osaka 565-0871 (JP); SUGA, Misao, Osaka-shi, Osaka 541-8505 (JP); FUJITA, Takuya, Osaka-shi, Osaka 541-8505 (JP)
(74) Representative: Hoffmann Eitle
(86) International application number: PCT/JP2023/040020
(87) International publication number: WO 2024/101345

(57) **Abstract**

Provided is an agent for inhibiting abnormal protein aggregate accumulation or an agent for inhibiting the uptake of an abnormal protein into a neuron, particularly an agent for preventing or treating a disease associated with accumulation of abnormal protein aggregates, in which the agent contains an RGMa inhibiting substance.

## Description

### TECHNICAL FIELD

The present invention relates to an agent for inhibiting abnormal protein aggregate accumulation comprising an RGMa inhibiting substance, particularly to an agent for preventing or treating frontotemporal lobar degeneration.

### BACKGROUND ART

In a cell, a misfolded protein (a protein not having a correct conformation) is rapidly degraded and removed, whereby the quality of proteins is controlled. However, if a genetic factor or an environmental factor (aging, inflammation, oxidative stress, or the like) distorts the protein quality control system, harmful protein aggregates accumulate in cells, causing various neurodegenerative diseases (Non-Patent Literature 1). In addition, misfolded protein aggregates spread between adjacent cells or to separate regions in the brain via the cerebrospinal fluid or the like, and thus, aggregate lesions expand (Non-Patent Literature 2).

Ubiquitin is a tag that mark misfolded proteins, and harmful protein aggregates (the aggregates of TDP-43, Tau, α-Synuclein, and SOD1, and the like) that cause neurodegeneration are also ubiquitinated (Non-Patent Literature 1). It has been reported that, ubiquitin-positive protein aggregates and inclusion bodies are detected in the autopsied brains of patients with various neurodegenerative diseases (Non-Patent Literature 3).

Frontotemporal dementia (FTD) is a group of neurodegenerative diseases that develop a noticeable behavior disorder, psychiatric symptom, language disorder, or the like owing to the progressive localized neurodegeneration and atrophy of the frontal lobe and anterior temporal lobe of the brain, (Non-Patent Literature 4). FTD is roughly classified into behavioral variant FTD (bvFTD), nonfluent-variant primary progressive aphasia (nfvPPA; progressive nonfluent aphasia, PNFA), and semantic-variant primary progressive aphasia (semantic-variant PPA, svPPA; semantic dementia, SD) according to clinical symptoms (Non-Patent Literature 4). FTD accompanied by a motor neuron disease is called FTD-MND. bvFTD is the most frequent subtype, and accounts for approximately 50% of the FTD cases. Typically, bvFTD is developed in an adult less than 65 years old, and characterized by a severe impairment in behavior, personality, and cognition.

Pathologically, FTD is frontotemporal lobar degeneration (FTLD) in which specific proteins aggregate in neurons or glia, causing inclusion bodies to accumulate. As main components of the inclusion bodies, tau protein, transactive response DNA binding protein of 43 kDa (TDP-43) protein, and fused in sarcoma (FUS) protein have been identified, and are used for pathological classification (Non-Patent Literature 5). A group in which tau protein is recognized in the proteins accumulated in an intracellular inclusion body is classified as FTLD-tau. It is known that the tau protein accumulated in the brain of a patient with FTLD-tau is posttranslationally modified variously by hyperphosphorylation, fragmentation, and ubiquitination (Non-Patent Literature 6). The tau protein-negative type is further subdivided, and for the TDP-43 protein-positive FTLD-TDP, FUS protein-positive FTLD-FUS, and the like, ubiquitin-positivity is included in the classification criterion (Non-Patent Literature 5). There is a report on a clinicopathologic correlation, the report stating that, in clinical diagnoses, half or more than half of bvFTD cases were FTLD-TDP, approximately 70% of nfvPPA (PNFA) cases were FTLD-tau, and approximately 80% of svPPA (SD) cases were FTLD-TDP (Non-Patent Literature 7). If a clinical diagnosis is FTD, there is an extremely high possibility that ubiquitinated protein aggregates are in the background pathology.

RGM (repulsive guidance molecule) is a membrane protein that has been initially identified as an axon guidance molecule in the visual system (see Non-Patent Literature 8). RGM family includes three members called RGMa, RGMb, and RGMc (Non-Patent Literature 9). At least RGMa and RGMb are known to work in the same signaling mechanism (Non-Patent Literature 10). RGMc plays an important role in iron metabolisms.

Subsequent studies have revealed that RGM functions to control, for example, axon guidance and laminar formation in Xenopus and chick embryos, and cephalic neural tube closure in mouse embryos (Non-Patent Literature 11). Patent Literature 1 discloses an axon regeneration promoting agent containing an anti-RGM neutralizing antibody as an active ingredient.

In addition to its functions in developmental stages, RGMa is expressed again after central nervous system injuries in an adult human and rat. Further, inhibition of RGMa in rat promotes axon growth after spinal cord injuries and facilitates functional recovery (Non-Patent Literature 12). From these facts, RGMa is considered as an inhibitor of axon regeneration after central nervous system injuries. Specific examples of antibodies to neutralize RGMa include those described in Patent Literature 2 (such as 5F9, and 8D1), Patent Literature 3 (such as AE12-1, an AE12-1Y), and Patent Literature 4 (such as r116A3, r70E4, r116A3C, and rH116A3).

As described above, roles of RGMa in central nervous system injuries have already been revealed, but involvement of RGMa particularly in the inhibition of formation of ubiquitinated aggregates (positive inclusion bodies) has not been identified, and no such therapeutic agent has been known.

### CITATION LIST

### Patent Literature

[Patent Literature 1] International Publication No. WO2005/087268
[Patent Literature 2] International Publication No. WO2009/106356
[Patent Literature 3] International Publication No. WO2013/112922
[Patent Literature 4] International Publication No. WO2016/175236

### Non-Patent Literature

[Non-Patent Literature 1] Watanabe Y, Taguchi K, Tanaka M. Ubiquitin, Autophagy and Neurodegenerative Diseases. Cells. 2020; 9(9):2022
[Non-Patent Literature 2] Soto C, Pritzkow S. Protein misfolding, aggregation, and conformational strains in neurodegenerative diseases. Nat Neurosci. 2018 Oct; 21(10): 1332-1340
[Non-Patent Literature 3] Chung KK, Dawson VL, Dawson TM. The role of the ubiquitin-proteasomal pathway in Parkinson's disease and other neurodegenerative disorders. Trends Neurosci. 2001 Nov;24(11 Suppl): 7-14
[Non-Patent Literature 4] Frontotemporal dementia. Bang J, Spina S, Miller BL. Lancet. 2015 Oct 24;386(10004):1672-82. doi: 10.1016/S0140-6736(15)00461-4
[Non-Patent Literature 5] Review: an update on clinical, genetic and pathological aspects of frontotemporal lobar degenerations. Lashley T, Rohrer JD, Mead S, Revesz T. Neuropathol Appl Neurobiol. 2015 Dec;41(7):858-81. doi: 10.1111/nan.12250. Epub 2015 Jul 6. PMID: 26041104
[Non-Patent Literature 6] Mandelkow EM, Mandelkow E. Biochemistry and cell biology of tau protein in neurofibrillary degeneration. Cold Spring Harb Perspect Med 2012; 2: a006247
[Non-Patent Literature 7] Therapeutic and diagnostic challenges for frontotemporal dementia. D'Alton S, Lewis J. Front Aging Neurosci. 2014 Aug 19;6:204. doi: 10.3389/fnagi.2014.00204. eCollection 2014. PMID: 25191265
[Non-Patent Literature 8] Stahl, B., Muller, B., von Boxberg, Y., Cox, E.C. & Bonhoeffer, F. Biochemical characterization of a putative axonal guidance molecule of the chick visual system. Neuron 5, 735-743 (1990)
[Non-Patent Literature 9] Mueller et al., Philos. Trans. R. Soc. Lond. B Biol. Sci., 361: 1513-29, 2006
[Non-Patent Literature 10] Liu, X., Hashimoto, M., Horii, H., Yamaguchi, A., Naito, K. and Yamashita, T. Repulsive guidance molecule b inhibits neurite growth and is increased after spinal cord injury. Biochem. Biophys. Res. Commun. 382, 795-800 (2009)
[Non-Patent Literature 11] Yamashita, T., Mueller, B.K. & Hata, K. Neogenin and repulsive guidance molecule signaling in the central nervous system. Curr. Opin. Neurobiol.17, 29-34 (2007)
[Non-Patent Literature 12] Hata, K. et al. RGMa inhibition promotes axonal growth and recovery after spinal cord injury. J. Cell Biol. 173, 47-58 (2006)

### SUMMARY OF INVENTION

### Problem to be solved by the invention

An object of the present invention is to provide a drug that has the effect of inhibiting the accumulation of abnormal protein aggregates, and that is effective against a disease associated with accumulation of abnormal protein aggregates.

### Means to solve the problem

The present inventors have intensively studied to achieve the object, and come to complete the present invention through the discovery that an RGMa inhibiting substance, particularly an anti-RGMa neutralizing antibody has the effect of inhibiting the accumulation of abnormal protein aggregates, and is expected to have a therapeutic effect against a disease associated with accumulation of abnormal protein aggregates.

In other words, the present invention is as follows.

1. An agent for inhibiting abnormal protein aggregate accumulation, comprising an RGMa inhibiting substance as an active ingredient.
2. An agent for inhibiting the uptake of an abnormal protein into a neuron, comprising an RGMa inhibiting substance as an active ingredient.
3. The agent according to 1 or 2 above, wherein the agent for inhibiting abnormal protein aggregate accumulation or the agent for inhibiting the uptake of an abnormal protein into a neuron is an agent for preventing or treating a disease associated with accumulation of abnormal protein aggregates.
4. The agent according to 3 above, wherein the disease associated with accumulation of abnormal protein aggregates is a disease selected from spinocerebellar degeneration, spinocerebellar ataxia, dentatorubropallidoluysian atrophy, fragile X-associated tremor/ataxia syndrome, spinobulbar muscular atrophy, fragile X syndrome, fragile XE syndrome, myotonic dystrophy (type 1), lethal insomnia, frontotemporal dementia or frontotemporal lobar degeneration, Pick disease, primary progressive aphasia, primary progressive nonfluent aphasia, progressive supranuclear palsy, corticobasal degeneration, argyrophilic grain dementia, globular glial tauopathy, and dementia with Lewy body.
5. The agent according to 3 or 4 above, wherein the disease associated with accumulation of abnormal protein aggregates is frontotemporal dementia (FTD) or frontotemporal lobar degeneration (FTLD).
6. The agent according to any one of 1 to 5 above, wherein the RGMa inhibiting substance is an anti-RGMa neutralizing antibody.
7. The agent according to 6 above, wherein the anti-RGMa neutralizing antibody is a humanized antibody.
8. The agent according to 6 or 7 above, wherein the anti-RGMa neutralizing antibody is an antibody recognizing an amino acid sequence selected from SEQ ID NOS: 16, 36, 37, 38 and 39.
9. The agent according to any one of 6 to 8 above, wherein the anti-RGMa neutralizing antibody is an antibody selected from the following (a) to (l):
   (a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9, and an HCDR3 comprising the amino acid sequence represented by SEQ ID NO: 10;
   (b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15, and an HCDR3 comprising an amino acid sequence comprising SFG;
   (c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18, and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;
   (d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27, and an HCDR3 comprising the amino acid sequence represented by SEQ ID NO: 28;
   (e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
   (k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34; and
   (l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34.
10. A method of preventing or treating a disease associated with accumulation of abnormal protein aggregates, the method comprising administering an effective dose of an RGMa inhibiting substance to a mammal in need of treatment.
11. The preventive or therapeutic method according to 10 above, wherein the RGMa inhibiting substance is an anti-RGMa neutralizing antibody.
12. Use of an RGMa inhibiting substance in manufacture of an agent for preventing or treating a disease associated with accumulation of abnormal protein aggregates.
13. The use according to 12 above, wherein the RGMa inhibiting substance is an anti-RGMa neutralizing antibody.

### ADVANTAGEOUS EFFECTS OF INVENTION

According to the present invention, an RGMa inhibiting substance, particularly an anti-RGMa neutralizing antibody has the effect of inhibiting the accumulation of abnormal protein aggregates or the effect of inhibiting the uptake of an abnormal protein into a neuron, and is useful as an agent for preventing or treating a disease associated with accumulation of abnormal protein aggregates, for example, spinocerebellar degeneration, spinocerebellar ataxia, dentatorubropallidoluysian atrophy, fragile X-associated tremor/ataxia syndrome, spinobulbar muscular atrophy, fragile X syndrome, fragile XE syndrome, myotonic dystrophy (type 1), lethal insomnia, frontotemporal dementia or frontotemporal lobar degeneration, Pick disease, primary progressive aphasia, primary progressive nonfluent aphasia, progressive supranuclear palsy, corticobasal degeneration, argyrophilic grain dementia, globular glial tauopathy, or dementia with Lewy body.

### BRIEF DESCRIPTION OF THE DRAWINGS

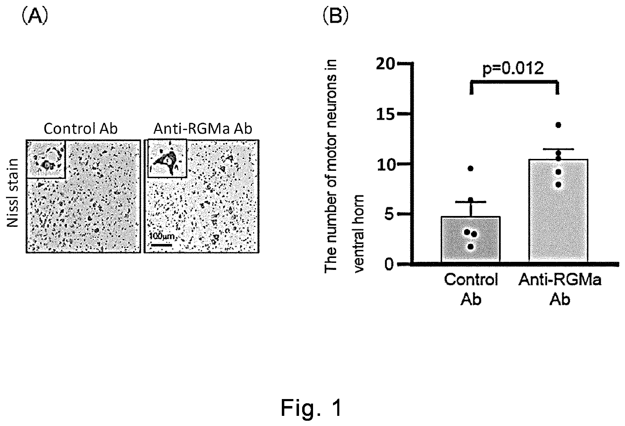

[FIG. 1] FIG. 1 is immunostaining images (photographs as a substitute for a drawing) and a graph showing that, in mSOD1 mice, an anti-RGMa neutralizing antibody (Anti-RGMa Ab, hereinafter, the notations in the drawing are synonymous) preserved the number of motor neurons with normal morphology. (A) of FIG. 1 shows representative images of motor neurons of the spinal cord of the mSOD1 mouse, in which the motor neurons were detected by Nissl staining. The number of ventral horn cells was reduced in mSOD1 mice treated with control antibody (Control Ab, hereinafter, the notations in the drawing are synonymous) compared with mSOD1 mice treated with anti-RGMa neutralizing antibody, and remaining ventral horn cells appeared atrophic in the mSOD1 mice treated with control antibody. (B) of FIG. 1 shows the quantitative analysis, neuron cell bodies that each had an area of more than 300 µm² were selected from 15 sections of each individual, were counted as motor neurons . Data are shown as mean ± standard error with individual value plots (the control antibody-teated group, n = 5; the anti-RGMa neutralizing antibody-treated group, n = 5). For statistical analysis, Student's t-test was usede

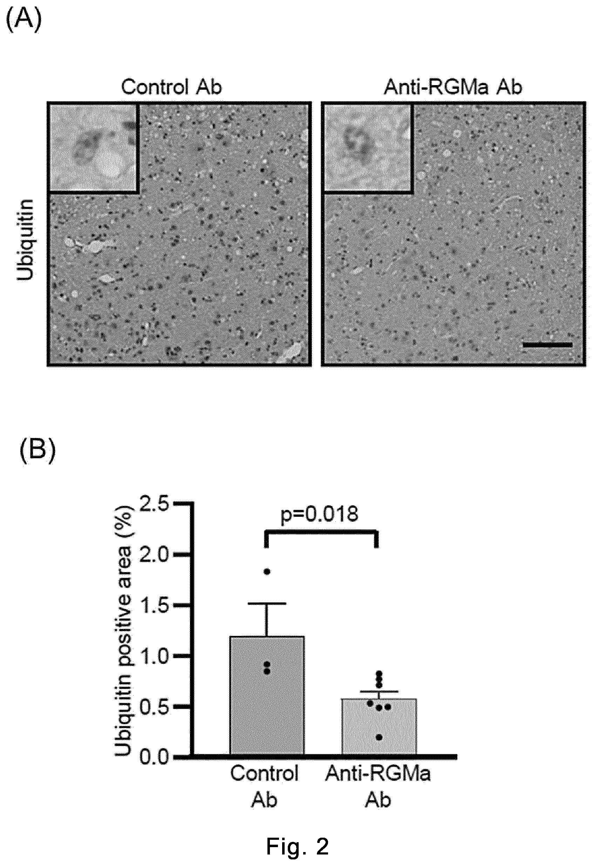

[FIG. 2] FIG. 2 is immunostaining images (photographs as a substitute for a drawing) of spinal cord pathology and a graph showing that, in the mSOD1 mice study, the administration of anti-RGMa neutralizing antibody decreased ubiquitin-positive protein aggregates. (A) of FIG. 2 shows representative images of motor neurons of the spinal cord of the mSOD1 mouse, in which the motor neurons were detected by ubiquitin staining (the scale bar is 300 µm). (B) of FIG. 2 shows the results of the quantitative analysis shown by the ratio of the ubiquitin-positive area to the area of the ventral horn of the spinal cord, and the mean ±standard error are shown with individual value plots (the control antibody-treated group, n = 3; the anti-RGMa neutralizing antibody-treated group, n = 7). For statistical analysis, Mann-Whitney U (MWU) test was used.

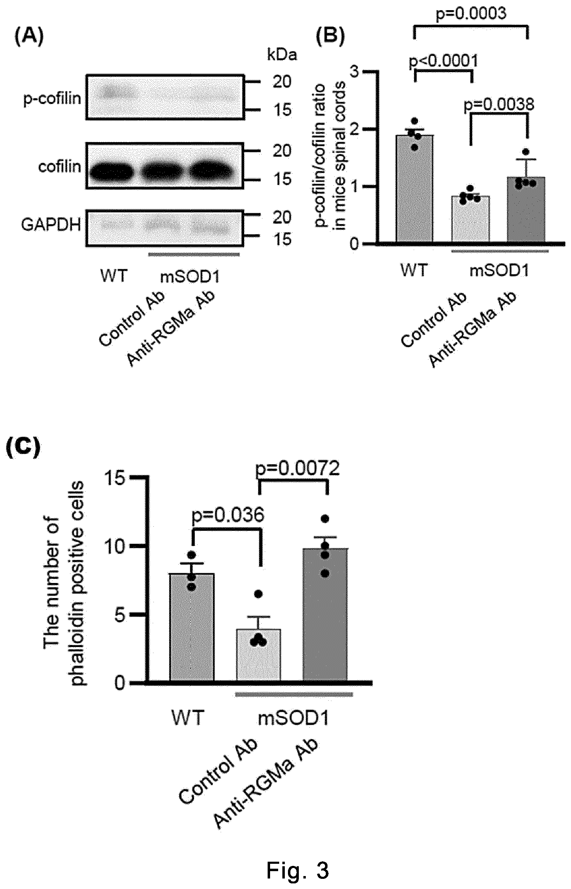

[FIG. 3] (A) and (B) of FIG. 3 show Western blotting images (photographs as a substitute for a drawing) and graphs showing that, the dephosphorylation of cofilin was increased in the spinal cord of mSOD1 mice, and the increase was inhibited by an anti-RGMa neutralizing antibody. By quantitative analysis, the ratio of phosphorylated cofilin to cofilin was determined. The mean ± standard error are shown with individual value plots (the wild type WT, n = 4; the control antibody-treated group, n = 5; the anti-RGMa neutralizing antibody-treated group , n = 5). For statistical analyses, one-way ANOVA and Tukey's multiple comparisons test were used (P-cofilin: phosphorylated cofilin). (C) of FIG. 3 shows a graph showing that, actin polymerization decreased in the spinal cord of mSOD1 mice, and the anti-RGMa neutralizing antibody inhibited a decrease in the actin polymerization. To detect F actin, phalloidin staining was used, and the number of phalloidin-positive motor neurons was counted. The mean ± standard error are shown together with individual value plots (the wild type WT, n = 3; the control antibody group, n = 4; the group to which the anti-RGMa neutralizing antibody was administered, n = 4). For statistical analyses, one-way ANOVA and Tukey's multiple comparisons test were used.

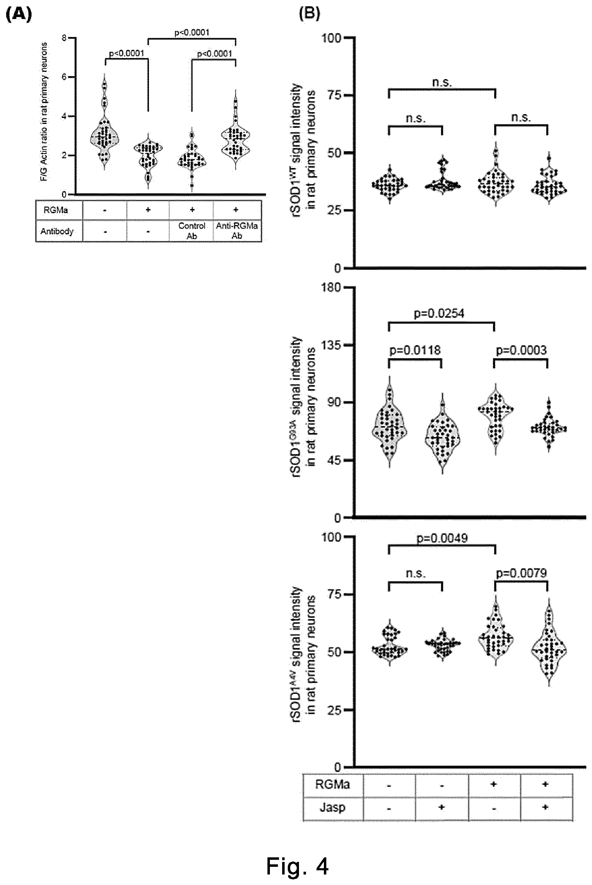

[FIG. 4] (A) of FIG. 4 shows a graph showing that, in the rat primary cortical neuron, RGMa decreased actin polymerization, and that the actin polymerization decreasing effect of the RGMa was inhibited by the anti-RGMa neutralizing antibody. From the images of double immunostaining of F-actin and G-actin, the respective staining intensities were quantitated to determine the ratio for each cell, which was used as an index of actin polymerization. With respect to each evaluation condition, a total of 36 cells from 3 wells were used for analysis. The mean ± standard error are shown. For statistical analyses, one-way ANOVA and Tukey's multiple comparisons test were used. (B) of FIG. 4 illustrates graphs showing that, in the rat primary cortical neuron, RGMa enhanced the entry of mutated SOD1 protein into the cells. Using an anti-FLAG antibody conjugated with FITC, SOD1 protein was revealed in immunostaining images, which were used to select FITC-positive cells, and the average signal intensity was quantitatively analyzed. With respect to each condition, a total of 36 cells selected from 3 wells were used for quantitation. The mean ± standard error are shown with the of individual plot of each cell. For statistical analyses, one-way ANOVA and Tukey's multiple comparisons test were used. (Jasp: Jasplakinolide)

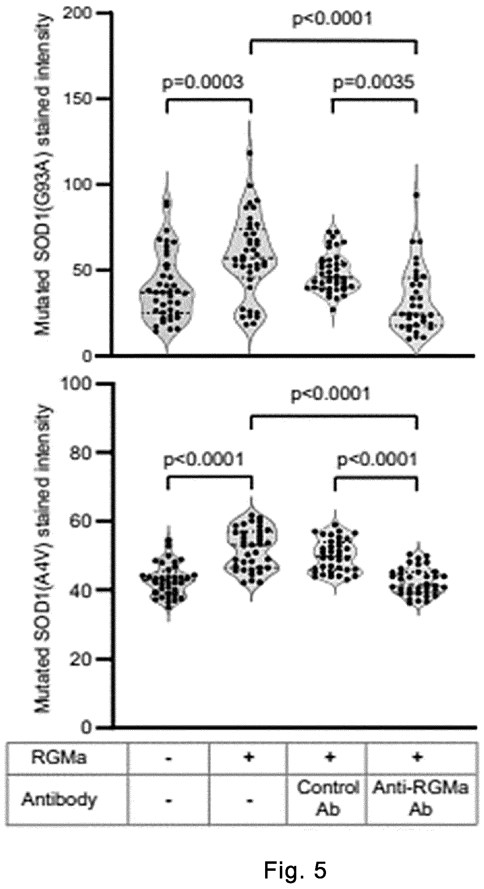

[FIG. 5] FIG. 5 is graphs showing that, in the rat primary cortical neuron, an increase caused by RGMa in the entry of a mutated SOD1 protein into cells was inhibited by the anti-RGMa neutralizing antibody. Using an anti-FLAG antibody conjugated with FITC, SOD1 protein was revealed in immunostaining images, which were used to select an FITC-positive cell, and the average signal intensity was quantitatively analyzed. With respect to each condition, a total of 36 cells selected from 3 wells were used for quantitation. The mean ± standard error are shown with the of individual plot of each cell. For statistical analyses, one-way ANOVA and Tukey's multiple comparisons test were used.

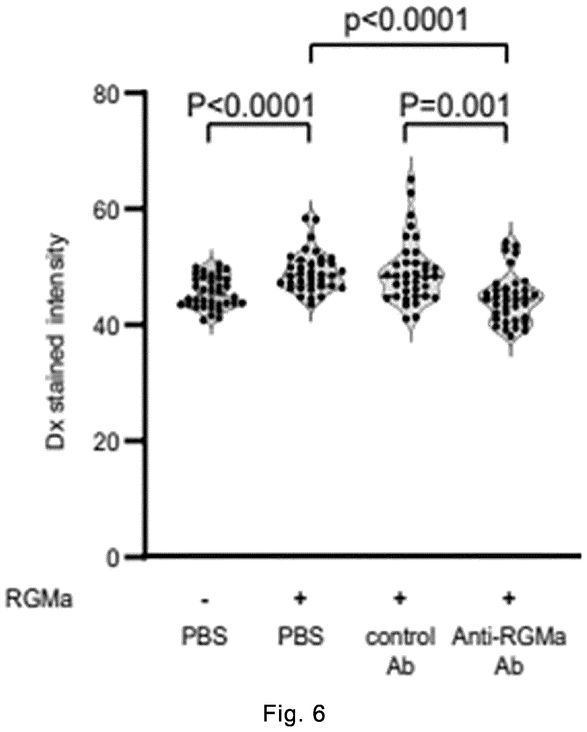

[FIG. 6] FIG. 6 is graphs showing that, in the rat primary cortical neuront, the RGMa enhanced the entry of dextran into cells, and the enhancement of entry was inhibited by the anti-RGMa neutralizing antibody. With respect to each condition, a total of 36 cells were selected from 3 wells, and the average fluorescence intensity showing the uptake of dextran into a cell was quantitatively analyzed. The mean ±standard error are shown with the individual plot of each cell. For statistical analyses, one-way ANOVA and Tukey's multiple comparisons test were used.

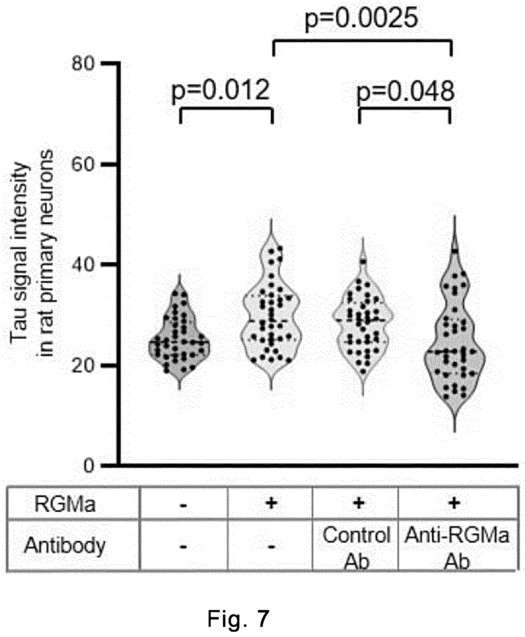

[FIG. 7] FIG. 7 is graphs showing that, in the rat primary cortical neuron, the RGMa increased the entry of human Tau protein into cells, and the increase of entry was inhibited by the anti-RGMa neutralizing antibody. Using Tau antibody and AlexaFluor (trademark) 594, Tau protein was revealed in immunostaining images, which were used to quantitatively analyze the average signal intensities. With respect to each condition, a total of 36 cells were selected from independent two wells. The mean ± standard error are shown with the individual plot of each cell. For statistical analyses, one-way ANOVA and Tukey's multiple comparisons test were used.

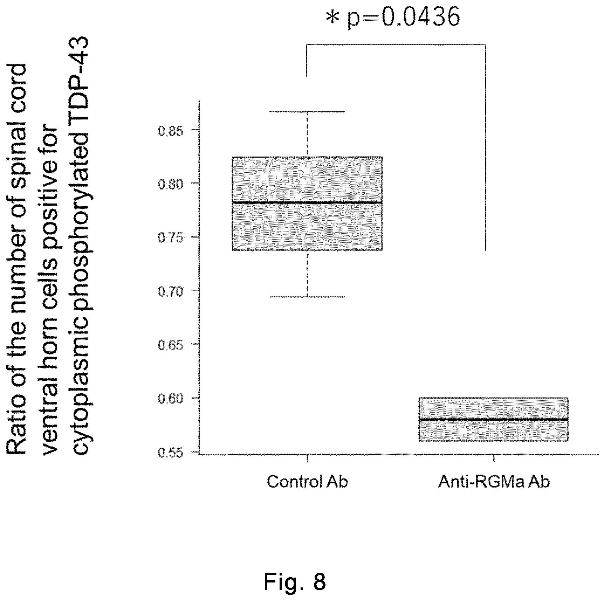

[FIG. 8] FIG. 8 shows that anti-RGMa neutralizing antibody treatment reduced the aggregation of phosphorylated TDP-43 in mPFN1 mouse study. Results of quantitative analysis are expressed in vertical axis as the ratio of the number of spinal cord ventral horn cells positive for cytoplasmic phosphorylated TDP-43 to the total number of spinal cord ventral horn cells and are shown as mean ± standard error (n=3 in the control antibody-treated group and n=2 in the anti-RGMa neutralizing antibody-treated group). Welch's t-test was used for statistical analysis.

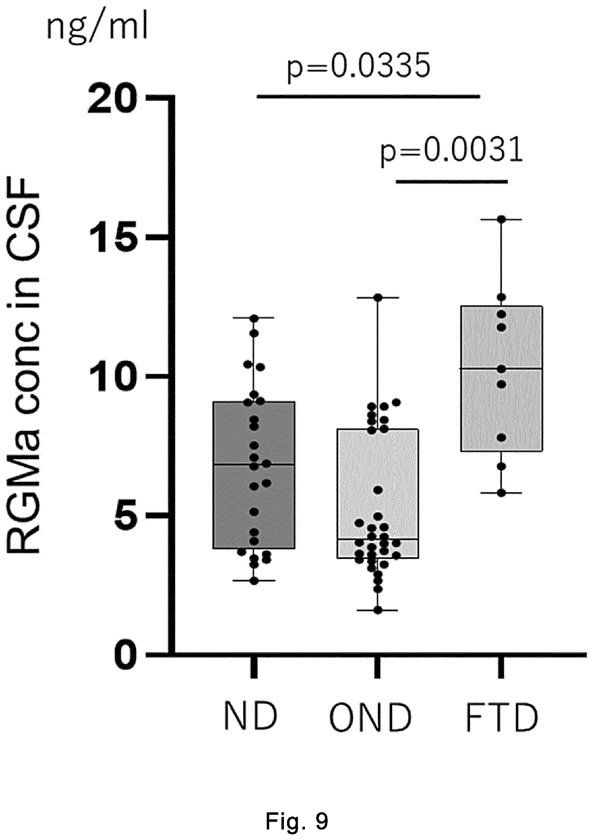

[FIG. 9] FIG. 9 is a graph showing that the RGMa concentration increased in the cerebrospinal fluid of an FTD patient. ANCOVA was used for statistical analysis. In the graph, ND and OND represent patients with non-neurodegenerative disorders and patients with other neurodegenerative disorders respectively.

### DETAILED DESCRIPTION OF THE INVENTION

The terms used in the present invention will be described below.

### [Neutralization]

The term "neutralization" as used herein refers to an action through which binding to a target of interest and inhibition of any function of the target can be made. For example, an RGMa inhibiting substance refers to a substance which binds to RGMa and consequently acts to inhibit the biological activity of RGMa.

### [Epitope]

As used herein, the term "epitope" includes a polypeptide determinant that can specifically bind to an immunoglobulin or a T-cell receptor. In some embodiments, an epitope can include a chemically active group on the surface of the molecule (for example, an amino acid, a sugar side chain, phosphoryl, or sulfonyl). In some embodiments, an epitope can have particular characteristics of three-dimensional structure and/or electric charge. Epitopes refer to regions in antigens, to which antibodies bind.

### [Isolated]

As used herein, the term "isolated" such as in isolated RGMa inhibiting substance (for example, antibody) means being identified, and separated and/or recovered from components in natural states. Impurities in natural states are substances that can interfere with the diagnostic or therapeutic use of the antibody, including enzymes, hormones and other proteinous or nonproteinous solutes. Generally, RGMa inhibiting substances or the like may be isolated through at least one purification step. RGMa inhibiting substances purified through at least one purification step can be referred to as "isolated RGMa inhibiting substances".

### [Antibody]

As used herein, the term "antibody" refers broadly to an immunoglobulin (Ig) molecule comprising four polypeptide chains, namely two heavy chains (H chains) and two light chains (L chains), that substantially retain the characteristics of an Ig molecule to bind to an epitope.

### [Human Antibody]

As used herein, the term "human antibody" refers to an antibody comprising light and heavy chains which are both derived from human immunoglobulins. Depending on the difference in the constant region of the heavy chain, human antibodies include IgG comprising a γ heavy chain (including IgG1, IgG2, IgG3 and IgG4); IgM comprising a µ heavy chain; IgA comprising an α heavy chain (including IgA1 and IgA2); IgD comprising a δ heavy chain; and IgE comprising an ε heavy chain. In principle, a light chain comprises either κ or λ chain.

### [Humanized Antibody]

As used herein, the term "humanized antibody" refers to an antibody comprising variable regions comprising complementarity determining regions from antibodies derived from non-human animals and framework regions derived from human antibodies, and constant regions derived from human antibodies.

### [Chimeric Antibody]

As used herein, the term "chimeric antibody" refers to an antibody in which the light chain, the heavy chain, or both comprise a non-human derived variable region and a human derived constant region.

### [Monospecific Antibody]

As used herein, the term "monospecific antibody" refers to an antibody comprising a single independent antigen recognition site having a single antigen specificity. For example, a monospecific antibody that recognizes RGMa may be referred herein to as an RGMa-monospecific antibody.

### [Multispecific Antibody]

As used herein, the term "multispecific antibody" refers to antibodies comprising two or more independent antigen recognition sites having two or more different antigen specificities, including bispecific antibodies having two antigen specificities and trispecific antibodies having three antigen specificities.

### [Complementarity Determining Region (CDR)]

The term "complementarity determining region (CDR)" refers to a region forming an antigen binding site in a variable region of an immunoglobulin molecule, which is also called a hypervariable region, and particularly refers to a portion in which the amino acid sequence changes greatly for each immunoglobulin molecule. As CDRs, light and heavy chains each have three CDRs. Three CDRs contained in a light chain may be referred to as LCDR1, LCDR2, and LCDR3, while three CDRs contained in a heavy chain may be referred to as HCDR1, HCDR2, and HCDR3. For example, CDRs of an immunoglobulin molecule are assigned according to the Kabat numbering system (Kabat et al., 1987 and 1991, Sequences of Proteins of Immunological Interest, US Department of Health and Human Services, NIH, USA). Herein, the CDR sequences defined according to the Kabat numbering system are used, unless otherwise specified.

In addition, for the CDR sequences of SEQ ID NOS: 5 to 15, the CDR sequences defined according to the numbering system of IMGT (Lefranc M. -P., Immunology Today 18,509 (1997), Lefranc M. -P., The Immunologist, 7, 132-136 (1999), Lefranc, M. -P., Pommie, C, Ruiz, M., Giudicelli, V., Foulquier, E., Truong, L., Thouvenin-Contet, V. and Lefranc, Dev. Comp. Immunol, 27, 55-77 (2003)) are used. For those skilled in the art, it is obvious that the CDR sequences described according to the IMGT numbering system are replaced with the CDR sequences described according to any other numbering system such as the Kabat numbering system. Accordingly, an invention in which the CDR sequences described according to the IMGT numbering system is replaced with the CDR sequences described according to the Kabat numbering system is included in embodiments of the present invention. In addition, an invention in which the CDR sequences described according to the Kabat numbering system is replaced with the CDR sequences described according to the IMGT numbering system is included in embodiments of the present invention.

### [Effective Dose]

The term "effective dose" refers to a sufficient dose of a preventive or therapeutic agent to alleviate or ameliorate the severity and/or duration of a disorder, or one or more symptoms thereof; to prevent progression of the disorder; to reverse the disorder; to prevent the recurrence, onset, development, or progression of one or more symptoms associated with the disorder; to detect the disorder; or to enhance or improve one or more preventive or therapeutic effects of another therapy (for example, a preventive or therapeutic drug).

### [Percent (%) Identity of Amino Acid Sequence]

"Percent (%) identity" of the amino acid sequence of a candidate polypeptide sequence, such as a variable region, with respect to the amino acid sequence of a reference polypeptide sequence is defined as a percent of the same amino acid residues in the candidate sequence as the amino acid residues in the particular reference polypeptide sequence, which percent is obtained by arranging the sequences and introducing gaps if necessary to obtain maximal % identity, without considering any conservative substitutions as part of the sequence identity. Alignment for determination of % identity can be accomplished by using various methods within the skill of the art, for example, using a publicly available computer software, such as BLAST, BLAST-2, ALIGN, or Megalign (DNASTAR) software. Those skilled in the art can determine appropriate parameters for sequence alignment, including algorithm needed to achieve maximal alignment over the full length of the sequence to be compared. However, for the purposes herein, values of % identity are obtained in pairwise alignment using a computer program for comparing sequences, BLAST.

When BLAST is used in comparison of amino acid sequences, the % identity of a given amino acid sequence A to a given amino acid sequence B is calculated as follows:
a fraction X/Y multiplied by 100
where X is the number of amino acid residues scored as identical in a programmed alignment of A and B by the sequence alignment program Blast, and Y is the total number of amino acid residues in B. It will be understood that when the lengths of amino acid sequences A and B are different, the % identities of A to B and of B to A are different. Unless stated otherwise, all % identity values herein are obtained using a computer program BLAST, as described in the immediately preceding paragraph.

### [Conservative Substitution]

"Conservative substitution" means replacement of an amino acid residue with another chemically similar amino acid residue so as not to substantially alter the activity of the peptide. Examples thereof include a case where a hydrophobic residue is substituted with another hydrophobic residue, and a case where a polar residue is substituted with another polar residue having the same charge. Examples of functionally similar amino acids eligible for such substitution include, as for non-polar (hydrophobic) amino acids, alanine, valine, isoleucine, leucine, proline, tryptophan, phenylalanine, and methionine. As for polar (neutral) amino acids, they include glycine, serine, threonine, tyrosine, glutamine, asparagine, and cysteine. As for positively charged (basic) amino acids, they include arginine, histidine, and lysine. As for negatively charged (acidic) amino acids, they include aspartic acid, and glutamic acid.

### [Treatment]

A "treatment" includes any treatment of a disease in a therapeutic objective, preferably a mammal, and especially a human, and includes prevention of progression of a disease or symptom so as to extinguish, heal, alleviate, or mitigate such a disease and symptom.

### [Prevention]

The term "prevention" includes prevention or inhibition of the onset of the above diseases in a therapeutic objective, preferably a mammal, and especially a human. Furthermore, "prevention" in the present invention includes "recurrence prevention" which prevents recurrence of the above-mentioned diseases in which remission and recurrence are repeated in a therapeutic objective, preferably a mammal, and especially a human.

Embodiments of the present invention will be described in detail below.

The present invention provides an agent for preventing or treating a disease associated with accumulation of abnormal protein aggregates, wherein the agent has the effect of inhibiting the accumulation of abnormal protein aggregates, wherein the effect is a novel use of an RGMa inhibiting substance.

Furthermore, the present invention provides a method of preventing or treating a disease associated with accumulation of abnormal protein aggregates, the method comprising a step of administering a preventive or therapeutic agent to a mammal in need of treatment, wherein the agent comprises an effective dose of an RGMa inhibiting substance.

### <RGMa inhibiting substance>

There is no particular restriction on the RGMa inhibiting substance of the present invention, insofar as it is a substance that acts on RGMa itself to inhibit or reduce the activity of RGMa (hereinafter sometimes referred to simply as "RGMa activity"). For example, a substance having an activity that directly inhibits (or reduces) the RGMa activity by binding to the RGMa, or an activity that indirectly inhibits (or reduces) the RGMa activity by inhibiting the binding of an RGMa to a receptor (e.g., the compound, antibody, etc. described below) is referred to as the RGMa inhibiting substance of the present invention.

The RGMa inhibiting substance of this invention may also be a substance that inhibits the expression of RGMa, for example, a substance that inhibits the expression of RGMa to inhibit (reduces) the RGMa activity (e.g., a nucleic acid molecule described below) is also included in the RGMa inhibiting substance of the present invention.

RGMa is identified as a protein that inhibits neurite outgrowth in the central nervous system. A human RGMa protein is biosynthesized as a precursor protein comprising 450 amino acids as shown in SEQ ID NO: 1. The signal peptide from Met 1 to Pro 47 present at the N terminus (which refers to the peptide from the first methionine residue to the 47th proline residue from the N-terminal side, and is hereafter described as above) is removed. Then, the peptide bond between Asp 168 and Pro 169 is cleaved to generate an N-terminal domain. In the C-terminal fragment from Pro 169, the peptide from Ala 425 to Cys 450 at the C terminus is removed so that Ala 424 becomes the C terminus. Then, a GPI anchor is added to the C-terminal carboxyl group of Ala 424 to generate a C-terminal domain. The human RGMa protein is expressed on the cell membrane via a GPI anchor as a mature protein in which the N-terminal domain (Cys 48 to Asp 168) and the C-terminal domain (Pro 169 to Ala 424) are linked by a disulfide bond.

RGMa in the present invention may be derived from any animals. Preferably, RGMa is human RGMa. A human RGMa precursor protein comprises the amino acid sequence shown in SEQ ID NO: 1 in Sequence Listing. A mouse RGMa precursor protein comprises the amino acid sequence shown in SEQ ID NO: 2 in Sequence Listing, while a rat RGMa precursor protein comprises the amino acid sequence shown in SEQ ID NO: 3 in Sequence Listing. However, removal of the C-terminal peptides results in mature proteins having the same amino acid sequence.

RGMa genes include, but are not limited to, a human RGMa gene comprising the nucleotide sequence shown in SEQ ID NO: 4. Nucleotide sequences of RGM genes derived from various organisms can be easily obtained from known databases (for example, GenBank).

Specific examples of the RGMa inhibiting substance of the present invention include low molecular weight compounds, anti-RGMa neutralizing antibodies, and functionally modified antibodies thereof, conjugated antibodies thereof, and antigen-binding fragments thereof, as well as an siRNA (short interfering RNA), an shRNA (short hairpin RNA), and an antisense oligonucleotide, which are nucleic acid molecules of RGMa. Among these RGMa inhibiting substances, an anti-RGMa neutralizing antibody, a functionally modified antibody thereof, a conjugated antibody thereof, and an antigen-binding fragment thereof are preferable, an anti-RGMa neutralizing antibody, and an antigen-binding fragment thereof are more preferable, and an anti-RGMa neutralizing antibody is especially preferable.

### <Anti-RGMa Neutralizing Antibody>

According to the present invention, the anti-RGMa neutralizing antibody may be an antibody that binds to RGMa to neutralize the RGMa activity, and may be a polyclonal, or monoclonal antibody. A monoclonal antibody is more preferable according to the present invention. The anti-RGMa-neutralizing antibody of the present invention may be an RGMa-monospecific antibody, or a multispecific antibody that recognizes RGMa and a plurality of other antigens. An RGMa-monospecific antibody is more preferable.

Specifically, the epitopes in human RGMa are preferably one or more of SEQ ID NO: 16 (amino acid numbers 47 to 69 in SEQ ID NO: 1), SEQ ID NO: 36 (amino acid numbers 298 to 311 in SEQ ID NO: 1), SEQ ID NO: 37 (amino acid numbers 322 to 335 in SEQ ID NO: 1), SEQ ID NO: 38 (amino acid numbers 349 to 359 in SEQ ID NO: 1), and SEQ ID NO: 39 (amino acid numbers 367 to 377 in SEQ ID NO: 1); more preferably a combination of SEQ ID NOS: 36 and 37;, and particularly preferably a combination of SEQ ID NOS: 36, 37 and 39.

The anti-RGMa neutralizing antibodies of the present invention include polyclonal or monoclonal antibodies obtained by immunizing mammals such as mice with an antigen which is an RGMa protein or a partial fragment thereof (for example, epitope fragment described above); chimeric antibodies and humanized antibodies produced by gene recombination technology; and human antibodies produced, for example, by a transgenic animal producing human antibody. When the antibody of the present invention is administered to a human as a medicine, the antibody is desirably a humanized antibody or a human antibody from the viewpoint of side effects.

Specific examples of the anti-RGMa neutralizing antibody of the present invention include the following antibodies (a) to (1). As the respective production methods therefor, the methods described in Patent Literature 2 to 4 may be used.

Examples thereof include antibodies to be selected from: (a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6 and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9 and an HCDR3 comprising the amino acid sequence represented by SEQ ID NO: 10 (the anti-RGMa neutralizing antibody also includes antibodies whose epitopes are SEQ ID NOS: 36, 37 and 39);
SEQ ID NO: 5: QDISSY
SEQ ID NO: 6: YTS (qualifier: mol_type = protein; organism = Mus musculus)
SEQ ID NO: 7: QQLNTLPWT
SEQ ID NO: 8: GFTFSDAW
SEQ ID NO: 9: IRSKANNHAT
SEQ ID NO: 10: TRRDGAY

(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12 and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15 and an HCDR3 comprising an amino acid sequence comprising AGS (the anti-RGMa neutralizing antibody also includes antibodies whose epitopes are SEQ ID NOS: 36, 37 and 38);
SEQ ID NO: 11: QSLVHSNGNTY
SEQ ID NO: 12: KVS (qualifier: mol_type = protein; organism = Mus musculus)
SEQ ID NO: 13: SQSTHVPYT
SEQ ID NO: 14: GYSITTSYY
SEQ ID NO: 15: ISYDGTN

(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18 and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21 and an HCDR3 comprising the amino acid sequence represented by SEQ ID NO: 22;
SEQ ID NO: 17: RSSQSLEYSDGYTFLE
SEQ ID NO: 18: EVSNRFS
SEQ ID NO: 19: FQATHDPLT
SEQ ID NO: 20: NYGMN
SEQ ID NO: 21: MIYYDSSEKHYADSVKG
SEQ ID NO: 22: GTTPDY

(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24 and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27 and an HCDR3 comprising the amino acid sequence represented by SEQ ID NO: 28;
SEQ ID NO: 23: QASQDIDNYLA
SEQ ID NO: 24: GATNLAD
SEQ ID NO: 25: LQGYIPPRT
SEQ ID NO: 26: SYVMH
SEQ ID NO: 27: YIIPYNDNTKYNEKFKG
SEQ ID NO: 28: ARRNEYYGSSFFDY

(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is SEQ ID NO: 16);
SEQ ID NO: 29: TGTSSSVGDSIYVS
SEQ ID NO: 30: DVTKRPS
SEQ ID NO: 31: CSYAGTDTL
SEQ ID NO: 32: SHGIS
SEQ ID NO: 33: WISPYSGNTNYAQKLQG
SEQ ID NO: 34: VGSGPYYYMDV

(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is SEQ ID NO: 16);
SEQ ID NO: 29: TGTSSSVGDSIYVS
SEQ ID NO: 30: DVTKRPS
SEQ ID NO: 35: YSYAGTDTL
SEQ ID NO: 32: SHGIS
SEQ ID NO: 33: WISPYSGNTNYAQKLQG
SEQ ID NO: 34: VGSGPYYYMDV

(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is SEQ ID NO: 16);
SEQ ID NO: 29: TGTSSSVGDSIYVS
SEQ ID NO: 30: DVTKRPS
SEQ ID NO: 40: FSYAGTDTL
SEQ ID NO: 32: SHGIS
SEQ ID NO: 33: WISPYSGNTNYAQKLQG
SEQ ID NO: 34: VGSGPYYYMDV

(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is SEQ ID NO: 16);
SEQ ID NO: 29: TGTSSSVGDSIYVS
SEQ ID NO: 30: DVTKRPS
SEQ ID NO: 41: HSYAGTDTL
SEQ ID NO: 32: SHGIS
SEQ ID NO: 33: WISPYSGNTNYAQKLQG
SEQ ID NO: 34: VGSGPYYYMDV

(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is SEQ ID NO: 16);
SEQ ID NO: 29: TGTSSSVGDSIYVS
SEQ ID NO: 30: DVTKRPS
SEQ ID NO: 42: LSYAGTDTL
SEQ ID NO: 32: SHGIS
SEQ ID NO: 33: WISPYSGNTNYAQKLQG
SEQ ID NO: 34: VGSGPYYYMDV

(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is SEQ ID NO: 16);
SEQ ID NO: 29: TGTSSSVGDSIYVS
SEQ ID NO: 30: DVTKRPS
SEQ ID NO: 43: VSYAGTDTL
SEQ ID NO: 32: SHGIS
SEQ ID NO: 33: WISPYSGNTNYAQKLQG
SEQ ID NO: 34: VGSGPYYYMDV

(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is SEQ ID NO: 16); and
SEQ ID NO: 29: TGTSSSVGDSIYVS
SEQ ID NO: 30: DVTKRPS
SEQ ID NO: 44: ISYAGTDTL
SEQ ID NO: 32: SHGIS
SEQ ID NO: 33: WISPYSGNTNYAQKLQG
SEQ ID NO: 34: VGSGPYYYMDV

(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34 (the anti-RGMa neutralizing antibody also includes an antibody whose epitope is SEQ ID NO: 16); and
SEQ ID NO: 29: TGTSSSVGDSIYVS
SEQ ID NO: 30: DVTKRPS
SEQ ID NO: 45: KSYAGTDTL
SEQ ID NO: 32: SHGIS
SEQ ID NO: 33: WISPYSGNTNYAQKLQG
SEQ ID NO: 34: VGSGPYYYMDV
Among these, the antibody according to (a) is particularly preferable.

In addition, examples of an anti-RGMa neutralizing antibody according to the present invention include antibodies described in WO2016/175236, WO2009/106356, and WO2013/112922, and the antibodies described in these bulletins are included in the anti-RGMa neutralizing antibodies according to the present invention. Specific examples of an anti-RGMa neutralizing antibody according to the present invention include an antibody selected from the following (a') to (i').

(a') an anti-RGMa neutralizing antibody comprising a light chain variable region comprising the amino acid sequence represented by SEQ ID NO: 46 and a heavy chain variable region comprising the amino acid sequence represented by SEQ ID NO: 47;
SEQ ID NO: 46: 
DIQMTQSPSSVSASVGDRVTITCRASQDISSYLNWYQQKPGKAPKLLIYYTSRLH
SGVPSRFSGSGSGTDFTLTISSLQPEDFASYFCQQLNTLPWTFGGGTKVEME
SEQ ID NO: 47:

EVQLVESGGGLVQPGRSLRLSCTASGFTFSDAWMDWVRQAPGKGLEWVAEIRS
KANNHATYYAESVKGRFTISRDDSKSIVYLQMNSLRTEDTALYYCTRRDGAYW
GKGTTVTVSS

(b') an anti-RGMa neutralizing antibody comprising a light chain comprising the amino acid sequence represented by SEQ ID NO: 48 and a heavy chain comprising the amino acid sequence represented by SEQ ID NO: 49;
SEQ ID NO: 48: 
DIQMTQSPSSVSASVGDRVTITCRASQDISSYLNWYQQKPGKAPKLLIYYTSRLH
SGVPSRFSGSGSGTDFTLTISSLQPEDFASYFCQQLNTLPWTFGGGTKVEMERTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 49:

EVQLVESGGGLVQPGRSLRLSCTASGFTFSDAWMDWVRQAPGKGLEWVAEIRS
KANNHATYYAESVKGRFTISRDDSKSIVYLQMNSLRTEDTALYYCTRRDGAYW
GKGTTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIA

VEWESNGQPENNYKTTPPVLDSDGSFFLYSKLTVDKSRWQQGNVFSCSVMHEA
LHNHYTQKSLSLSPGK

(c') an anti-RGMa neutralizing antibody comprising a light chain variable region comprising the amino acid sequence represented by SEQ ID NO: 50 and a heavy chain variable region comprising the amino acid sequence represented by SEQ ID NO: 51;
SEQ ID NO: 50: 
DVVMTQTPLSLPVSLGDQASISCRSSQSLVHSNGNTYLHWYLQRPGQSPKLLIY
KVSNRFSGVPDRFSGSGSGTDFTLKISRVEAEDLGLYFCSQSTHVPYTFGGGTKL
EIK
SEQ ID NO: 51:

DVKLQESGPGLVKPSQSLSLTCSVTGYSITTSYYWNWIRQFPGNKLEWMGYISY
DGTNNYNPSLKNRISITRDTSKNQFFLRLNSVTTEDTATYYCAGSFGYSQGTLVT
VSA

(d') an anti-RGMa neutralizing antibody comprising a light chain comprising the amino acid sequence represented by SEQ ID NO: 52 and a heavy chain comprising the amino acid sequence represented by SEQ ID NO: 53;
SEQ ID NO: 52: 
DVVMTQTPLSLPVSLGDQASISCRSSQSLVHSNGNTYLHWYLQRPGQSPKLLIY
KVSNRFSGVPDRFSGSGSGTDFTLKISRVEAEDLGLYFCSQSTHVPYTFGGGTKL
EIKRADAAPTVSIFPPSSEQLTSGGASVVCFLNNFYPKDINVKWKIDGSERQNGV
LNSWTDQDSKDSTYSMSSTLTLTKDEYERHNSYTCEATHKTSTSPIVKSFNRNEC
SEQ ID NO: 53:

DVKLQESGPGLVKPSQSLSLTCSVTGYSITTSYYWNWIRQFPGNKLEWMGYISY
DGTNNYNPSLKNRISITRDTSKNQFFLRLNSVTTEDTATYYCAGSFGYSQGTLVT
VSAAKTTPPSVYPLAPGCGDTTGSSVTLGCLVKGYFPESVTVTWNSGSLSSSVH
TFPALLQSGLYTMSSSVTVPSSTWPSQTVTCSVAHPASSTTVDKKLEPSGPISTINP
CPPCKECHKCPAPNLEGGPSVFIFPPNIKDVLMISLTPKVTCVVVDVSEDDPDVQI
SWFVNNVEVHTAQTQTHREDYNSTIRVVSTLPIQHQDWMSGKEFKCKVNNKD
LPSPIERTISKIKGLVRAPQVYILPPPAEQLSRKDVSLTCLVVGFNPGDISVEWTSN

GHTEENYKDTAPVLDSDGSYFIYSKLNMKTSKWEKTDSFSCNVRHEGLKNYYL
KKTISRSPGK

(e') an anti-RGMa neutralizing antibody comprising a light chain variable region comprising the amino acid sequence represented by SEQ ID NO: 54 and a heavy chain variable region comprising the amino acid sequence represented by SEQ ID NO: 55;
SEQ ID NO: 54: 
DVVLTQTPVSLSVTLGDQASMSCRSSQSLEYSDGYTFLEWFLQKPGQSPQLLIY
EVSNRFSGVPDRFIGSGSGTDFTLKISRVEPEDLGVYYCFQATHDPLTFGSGTKLE
IKR
SEQ ID NO: 55:

EVQLVESGGGLVQPGSSLKLSCVASGFTFSNYGMNWIRQAPKKGLEWIGMIYY
DSSEKHYADSVKGRFTISRDNSKNTLYLEMNSLRSEDTAIYYCAKGTTPDYWGQ
GVMVTVSS

(f') an anti-RGMa neutralizing antibody comprising a light chain variable region comprising the amino acid sequence represented by SEQ ID NO: 56 and a heavy chain variable region comprising the amino acid sequence represented by SEQ ID NO: 57;
SEQ ID NO: 56: 
DIQMTQSPASLSASLEEIVTITCQASQDIDNYLAWYHQKPGKSPRLLIYGATNLA
DGVPSRFSGSRSGTQFSLKINRLQIEDLGIYYCLQGYIPPRTFGGGTKLELKR
SEQ ID NO: 57:

EVQLQQSGPELVKPGTSVKMSCKTSGYTFTSYVMHWVKQKPGQGLEWIGYIIP
YNDNTKYNEKFKGKATLTSDKSSSTAYMELSSLTSEDSAVYYCARRNEYYGSSF
FDYWGQGTTLTVSS

(g') an anti-RGMa neutralizing antibody comprising a light chain variable region (λ chain) comprising the amino acid sequence represented by SEQ ID NO: 58 and a heavy chain variable region comprising the amino acid sequence represented by SEQ ID NO: 59;
SEQ ID NO: 58: 
QSALTQPRSVSGSPGQSVTISCTGTSSSVGDSIYVSWYQQHPGKAPKLMLYDVT
KRPSGVPDRFSGSKSGNTASLTISGLQAEDEADYYCCSYAGTDTLFGGGTKVTV
L
SEQ ID NO: 59:

EVQLVQSGAEVKKPGASVKVSCKASGYTFTSHGISWVRQAPGQGLDWMGWIS
PYSGNTNYAQKLQGRVTMTTDTSTSTAYMELSSLRSEDTAVYYCAR
VGSGPYYYMDVWGQGTLVTVSS

(h') an anti-RGMa neutralizing antibody comprising a light chain variable region comprising the amino acid sequence represented by SEQ ID NO: 60 and a heavy chain variable region comprising the amino acid sequence represented by SEQ ID NO: 59;
SEQ ID NO: 60: 
QSALTQPRSVSGSPGQSVTISCTGTSSSVGDSIYVSWYQQHPGKAPKLMLYDVT
KRPSGVPDRFSGSKSGNTASLTISGLQAEDEADYYCYSYAGTDTLFGGGTKVTV
L
SEQ ID NO: 59:

EVQLVQSGAEVKKPGASVKVSCKASGYTFTSHGISWVRQAPGQGLDWMGWIS
PYSGNTNYAQKLQGRVTMTTDTSTSTAYMELSSLRSEDTAVYYCAR
VGSGPYYYMDVWGQGTLVTVSS

(i') an anti-RGMa neutralizing antibody comprising a light chain comprising the amino acid sequence represented by SEQ ID NO: 61 and a heavy chain comprising the amino acid sequence represented by SEQ ID NO: 62.
SEQ ID NO: 61: 
QSALTQPRSVSGSPGQSVTISCTGTSSSVGDSIYVSWYQQHPGKAPKLMLYDVT
KRPSGVPDRFSGSKSGNTASLTISGLQAEDEADYYCYSYAGTDTLFGGGTKVTV
LGQPKAAPSVTLFPPSSEELQANKATLVCLISDFYPGAVTVAWKADSSPVKAGVE
TTTPSKQSNNKYAASSYLSLTPEQWKSHRSYSCQVTHEGSTVEKTVAPTECS
SEQ ID NO: 62:

EVQLVQSGAEVKKPGASVKVSCKASGYTFTSHGISWVRQAPGQGLDWMGWIS

PYSGNTNYAQKLQGRVTMTTDTSTSTAYMELSSLRSEDTAVYYCARVGSGPYY
YMDVWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPEAAGGPSVFLFPPKPKDQLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPSREEMTKNQVSLTCLVK
GFYPSDIAVEWESNGQPENNYKTTPPVLDSDGSFFLYSKLTVDKSRWQQGNVFS
CSVLHEALHNHYTQKSLSLSPGK

The anti-RGMa neutralizing antibodies of the present invention can be produced using production methods that are conventionally and commonly used. Antigens may be directly used for immunization, or may be used as a complex with a carrier protein. For preparing a complex of an antigen and a carrier protein, condensing agents such as glutaraldehyde, carbodiimides, and maleimide active esters can be used. Examples of the carrier protein include bovine serum albumin, thyroglobulin, hemocyanin, and KLH.

Examples of the mammal to be immunized include mice, rats, hamsters, guinea pigs, rabbits, cats, dogs, pigs, goats, horses and cattle. Examples of the inoculation method include subcutaneous, intramuscular and intraperitoneal administrations. When administered, antigens may be administered in mixture with complete or incomplete Freund's adjuvant, and are usually administered once every 2 to 5 weeks. Antibody-producing cells obtained from the spleen or lymph nodes of the immunized animals are fused with myeloma cells and isolated as hybridomas. As the myeloma cells, those derived from mammals such as mouse, rat, and human are used.

### <Polyclonal Antibody>

The polyclonal antibodies can be obtained, for example, from a serum obtained from an immunized animal which is the mammal as described above immunized with the antigen as described above, if necessary in combination with a Freund's adjuvant.

### <Monoclonal Antibody>

Specifically, the monoclonal antibodies can be obtained, for example, as follows. That is, the antigen as described above is used as an immunogen, and the immunogen is injected or transplanted one or several times in combination with a Freund's adjuvant, as necessary, to the mammal as described above subcutaneously, intramuscularly, intravenously, into a footpad, or intraperitoneally for immunization. Typically, the immunization is performed 1 to 4 times about every 1 to 14 days from the initial immunization, and after about 1 to 5 days from the final immunization, antibody-producing cells are obtained from the immunized mammal.

The monoclonal antibodies can be obtained by a method well known to a person skilled in the art (for example, "Current Protocols in Molecular Biology" (John Wiley & Sons (1987)), or Antibodies: A Laboratory Manual, Ed. Harlow and David Lane, Cold Spring Harbor Laboratory (1988)).

The "hybridomas" secreting monoclonal antibodies can be prepared according to the method of Köhler and Milstein, et al. (Nature, 256, 495, 1975) or a modified method based on it. That is, they are prepared by cell fusion between an antibody-producing cell included in the spleen etc. obtained from an immunized mammal, and a myeloma cell, which is not capable of producing an autoantibody, and derived from a mammal, preferably mouse, rat, or human.

Examples of the myeloma cell which can be used in the cell fusion include mouse-derived myelomas such as P3/X63-AG8.653 (653), P3/NSI/1-Ag4-1 (NS-1), P3/X63-Ag8.U1 (P3U1), SP2/0-Ag14 (Sp2/O, Sp2), PAI, F0 and BW5147; rat-derived myelomas such as 210RCY3-Ag.2.3.; and human-derived myelomas such as U-266AR1, GM1500-6TG-A1-2, UC729-6, CEM-AGR, D1R11, and CEM-T15.

Examples of the fusion promoter include polyethylene glycols. The cell fusion can usually be performed by a reaction in a temperature range from 20 to 40°C, preferably from 30 to 37°C, for about 1 to 10 minutes usually using a polyethylene glycol (with an average molecular weight from 1000 to 4000) with a concentration from about 20 to 50%, wherein the ratio of the number of antibody-producing cells to the number of myeloma cells is usually from about 1:1 to 10:1.

Screening of hybridoma clones producing the monoclonal antibodies can be carried out by culturing the hybridomas, for example, in a microtiter plate and assaying the culture supernatants in the wells for the reactivity against an immunogen by an immunochemical method such as ELISA.

In the screening of antibody-producing hybridomas, whether the antibody inhibits the RGMa activity in the present invention is also determined in addition to the binding assay with an RGMa protein. The screening methods allow for selection of the anti-RGMa neutralizing antibody of the present invention.

Clones can be further obtained from the wells containing hybridomas producing the desired antibodies by cloning with limiting dilution. Hybridomas are usually selected and grown in an animal cell culture medium containing 10 to 20% fetal bovine serum, supplemented with HAT (hypoxanthine, aminopterin and thymidine).

The monoclonal antibodies can be produced from the hybridomas by culturing the hybridomas *in vitro,* or growing them *in vivo*, for example, in ascitic fluids of mammals, such as mice and rats, and isolating the monoclonal antibodies from the resulting culture supernatants or the ascitic fluids of the mammals.

When cultured *in vitro,* it is possible to use a nutrient medium suitable for growing, maintaining, and conserving hybridomas corresponding to various conditions such as the characteristics of the cell species to be cultured, or the culturing method, and producing a monoclonal antibody in the culture supernatant. Examples of the nutrient medium may include a known nutrient medium, or a nutrient medium prepared from a basal medium.

Examples of the basal medium include low-calcium media such as Ham's F12 medium, MCDB 153 medium, and low-calcium MEM medium, and high-calcium media such as MCDB 104 medium, MEM medium, D-MEM medium, RPMI 1640 medium, ASF 104 medium, and RD medium. The basal medium can contain, for example, sera, hormones, cytokines and/or various inorganic or organic substances according to the purpose.

The monoclonal antibodies can be isolated and purified by, for example, subjecting the above-described culture supernatant or ascitic fluid to saturated ammonium sulfate, euglobulin precipitation, a caproic acid treatment, a caprylic acid treatment, an ion exchange chromatography (such as DEAE or DE52), or an affinity column chromatography, for example with an anti-immunoglobulin column or a protein A column. Specifically, the monoclonal antibodies may be purified by any method known as an immunoglobulin purification method, and the purification can be easily achieved by means such as ammonium sulfate fractionation, PEG fractionation, ethanol fractionation, a method utilizing an anion exchanger, and further an affinity chromatography using an RGMa protein.

The monoclonal antibodies can also be obtained by a phage display method. In a phage display method, phages selected from an optional phage antibody library are screened using a desired immunogen, and phages having a desired binding ability to the immunogen are selected. Next, the antibody-corresponding sequence contained in the phage is isolated or sequenced. Based on the information of the isolated sequence or the determined sequence by the sequencing, an expression vector comprising a nucleic acid molecule encoding the antibody or antigen binding domain is constructed. The expression vector is then transfected into a cell line, and the cell line can be cultured to produce a monoclonal antibody. When a human antibody library is used as the phage antibody library, a human antibody having a desired binding ability can be produced.

### <Nucleic Acid Molecule>

A nucleic acid molecule encoding an anti-RGMa neutralizing antibody of the present invention or an antigen-binding fragment thereof can be obtained, for example, by the following method. First, total RNA is prepared from a cell such as hybridoma using a commercially available RNA extraction kit, and subsequently cDNAs are synthesized with a reverse transcriptase using random primers and the like. Next, by a PCR method using, as primers, oligonucleotides of sequences respectively conserved in the variable regions in the known human antibody heavy chain and light chain genes, cDNAs encoding the antibody are amplified. Sequences encoding the constant regions can be obtained by amplification of known sequences by a PCR method. The nucleotide sequence of the DNA can be sequenced by a conventional method, for example, by incorporating it into a plasmid for sequencing.

Alternatively, a DNA encoding the monoclonal antibody of the present invention can also be obtained by chemically synthesizing sequences of the variable regions or parts thereof and joining them to sequences comprising the constant regions.

The nucleic acid molecule may encode all of the constant regions and the variable regions of heavy and light chains, or may encode only the variable regions of heavy and light chains. In the case of encoding all of the constant regions and the variable regions, the nucleotide sequences of the constant regions of heavy and light chains are preferably those described in Nucleic Acids Research, vol. 14, p1779, 1986; The Journal of Biological Chemistry, vol. 257, p1516, 1982; or Cell, vol. 22, p197, 1980.

### <Functionally Modified Antibody>

A functionally modified antibody of an anti-RGMa neutralizing antibody is prepared by the following method. For example, when an anti-RGMa neutralizing antibody under this application is produced using CHO cells as host cells, in which the α1,6-fucosyl transferase (FUT8) gene is destructed, the fucose content in the sugar chain is decreased, and an antibody with an increased cell killing function is obtained. Meanwhile, when the same is produced using CHO cells as host cells, in which the FUT8 gene is transduced an antibody with a weak cell killing function is obtained (International Publication No. WO2005/035586, International Publication No. WO2002/31140, and International Publication No. WO00/61739). Meanwhile, the complement activation function can be modulated by modifying the amino acid residues in the Fc region (U.S. Patent No. No. 6737056, U.S. Patent No. 7297775, and U.S. Patent No. 7317091). Further, when the variant of the Fc region with enhanced binding to FcRn, which is one of Fc receptors, is used, the half-life in blood can be prolonged (Hashiguchi Shuhei, et al, Seikagaku, 2010, Vol. 82 (8), p710). These functionally modified antibodies can be produced by genetic engineering.

### <Conjugated Antibody>

Examples of a modified molecule of the anti-RGMa neutralizing antibody of the present invention include a conjugated antibody. Examples of the conjugated antibody include a conjugated antibody in which an RGMa neutralizing antibody is bound chemically or by a genetic engineering technique to a functional molecule other than the present anti-RGMa neutralizing antibody such as nonpeptidic polymers such as polyethylene glycol (PEG), radioactive substances, toxins, low molecular weight compounds, cytokines, growth factors (such as TGF-β, NGF, and neurotrophin), albumins, enzymes, and other antibodies.

When PEG is bound as the functional molecule, PEG having, but without limitation, a molecular weight from 2,000 to 100,000 Da, more preferably from 10,000 to 50,000 Da can be used. PEG may be linear or branched. PEG can be bound to the N-terminal amino group in the amino acids of an anti-RGMa neutralizing antibody, etc., for example by using an NHS active group.

When a radioactive substance is used as the functional molecule, its examples include ¹³¹I, ¹²⁵I, ⁹⁰Y, ⁶⁴Cu, ⁹⁹Tc, ⁷⁷Lu, and ²¹¹At. The radioactive substance can be directly bound to the anti-RGMa neutralizing antibody by a chloramine-T method or the like.

When a toxin is used as the functional molecule, examples thereof include bacterial toxins (such as diphtheria toxin), phytotoxins (such as ricin), low molecular weight toxins (such as geldanamycin), maytansinoids and calicheamicins.

When a low molecular weight compound is used as the functional molecule, examples include daunomycin, doxorubicin, methotrexate, mitomycin, neocarzinostatin, vindesine, and fluorescent dyes such as FITC.

When an enzyme is used as the functional molecule, examples include luciferases (such as firefly luciferases, and bacterial luciferases; US Patent No. 4,737,456), malate dehydrogenases, ureases, peroxidases (such as horseradish peroxidase (HRPO)), alkaline phosphatases, β-galactosidases, glucoamylases, lysozymes, saccharide oxidases (such as glucose oxidase, galactose oxidase, and glucose-6-phosphate dehydrogenase), heterocyclic oxidases (such as uricase, and xanthine oxidase), lactoperoxidases, and microperoxidases.

Examples of linkers used for chemically bonding the toxin, low molecular weight compound, or enzyme include divalent radicals (such as alkylene, arylene, and heteroarylene), linkers represented by -(CR₂)ₙO(CR₂)ₙ- (wherein R is any substituent, and n is a positive integer), alkoxy repeating units (such as polyethyleneoxy, PEG, and polymethyleneoxy), alkylamino (such as polyethyleneamino, and Jeffamine (trademark)), and diacid esters and amides (including succinate, succinamide, diglycolate, malonate, and caproamide). Chemical modification methods for binding functional molecules have already been established in this field (D. J. King., Applications and Engineering of Monoclonal Antibodies., 1998, T.J. International Ltd, Monoclonal Antibody-Based Therapy of Cancer., 1998, Marcel Dekker Inc.; Chari, et al., Cancer Res., 1992 Vol. 152: 127; and Liu, et al., Proc Natl Acad Sci USA., 1996 Vol 93: 8681).

### <Antigen-binding Fragment>

In embodiments of the present invention, "antigen-binding fragments" of antibodies means partial regions having antigen binding properties derived from the antibodies as described above. Specific examples of the fragments include F(ab')₂, Fab', Fab, Fv (variable fragment of antibody), disulfide-linked Fv, single-chain antibodies (scFv), and polymers thereof. Examples of the antigen-binding fragments also include conjugated fragments that bind, chemically or by genetic engineering, functional molecules other than the present anti-RGMa neutralizing antibody, such as nonpeptidic polymers, e.g. polyethylene glycol (PEG), radioactive substances, toxins, low molecular weight compounds, cytokines, growth factors (e.g. TGF-β, NGF, and neurotrophin), albumins, enzymes, and other antibodies.

The terms "F(ab')₂" and "Fab" mean antibody fragments produced by treating an immunoglobulin with pepsin or papain which are proteases, namely produced by digestion at the upstream or downstream side of the disulfide bond existing between two heavy chains in the hinge region. For example, when IgG is treated with papain, it is cleaved at the upstream side of the disulfide bond existing between the two heavy chains in the hinge region to produce two homologous antibody fragments each comprising a light chain comprising a VL (light chain variable region) and a CL (light chain constant region) and a heavy chain fragment comprising a VH (heavy chain variable region) and a CHγ1 (γ1 region in the heavy chain constant region), in which the light chain and the heavy chain fragment are linked to each other via a disulfide bond at the C-terminal domain. Each of the two homologous antibody fragments is referred to as Fab. Meanwhile, when IgG is treated with pepsin, it is cleaved cleavage at the downstream side of the disulfide bond existing between the two heavy chains in the hinge region to produce an antibody fragment that is slightly larger than the two Fab linked to each other at the hinge region. This antibody fragment is referred to as F(ab')₂.

### <Chimeric Antibody>

In a preferred mode of the anti-RGMa neutralizing antibody of the present invention, there is a chimeric antibody. Examples of the "chimeric antibody" include those in which the variable regions are derived from immunoglobulins from non-human animals (such as mouse, rat, hamster and chicken) and the constant regions are derived from human immunoglobulins. The chimeric antibody can be prepared, for example, by immunizing a mouse with the antigen, cleaving out a variable region that binds to the antigen from the gene encoding the mouse monoclonal antibody, and combining the variable region with a constant region of an antibody derived from human bone marrow. The constant region derived from a human immunoglobulin has a unique amino acid sequence depending on each isotype, such as IgG (IgG1, IgG2, IgG3, or IgG4), IgM, IgA (IgA1, or IgA2), IgD, or IgE. The constant region of the recombinant chimeric antibody according to the present invention may be a constant region of a human immunoglobulin belonging to any of the isotypes. The constant region is preferably a constant region of human IgG. The chimeric antibody gene thus prepared can be used to prepare an expression vector. A host cell is transformed with the expression vector to obtain a transformed cell that produces the chimeric antibody. Subsequently, the transformed cell is cultured to obtain the desired chimeric antibody from the culture supernatant.

### <Humanized Antibody>

In another preferred mode of the anti-RGMa neutralizing antibody of the present invention, there is a humanized antibody. The "humanized antibody" according to the present invention is an antibody obtained by grafting only the DNA sequence of the antigen binding site (CDRs; complementarity determining regions) of an antibody derived from a nonhuman animal such as a mouse to a human antibody gene (CDR grafting). The humanized antibody can be prepared according to the methods described in, for example, Japanese Translated PCT Patent Application Laid-open No. 1992-506458 and Japanese Patent No. 2912618. Specifically, the humanized antibody comprises CDRs partly or wholly derived from monoclonal antibodies from non-human mammals (such as mouse, rat, and hamster); framework regions of variable regions derived from human immunoglobulins; and constant regions derived from human immunoglobulins.

The humanized antibody according to the present invention can be produced, for example, as described below. Needless to say, however, the production method is not limited thereto.

For example, a recombinant humanized antibody derived from a mouse monoclonal antibody can be produced by genetic engineering with reference to Japanese Translated PCT Patent Application Laid-open No. 1992-506458 and Japanese Laid-open Patent Application (Kokai) No. 1987-296890. Specifically, DNA encoding the region of CDRs of a mouse heavy chain and DNA encoding the region of CDRs of a mouse light chain are isolated from hybridomas producing a mouse monoclonal antibody. Further, a human heavy chain gene encoding the whole region other than CDRs of the human heavy chain and a human light chain gene encoding the whole region other than CDRs of the human light chain are isolated from a human immunoglobulin gene.

The isolated DNA encoding the region of CDRs of the mouse heavy chain is grafted to the human heavy chain gene, and the product is introduced into an appropriate expression vector so that it is expressible. Similarly, the DNA encoding the region of CDRs of the mouse light chain is grafted to the human light chain gene, and the product is introduced into another appropriate expression vector so that it is expressible. Alternatively, the human heavy and light chain genes to which mouse CDRs are grafted may be introduced into the same expression vector so that they are expressible. A host cell is transformed with the expression vector thus prepared to obtain a transformed cell producing the humanized antibody. Subsequently, the transformed cell is cultured to obtain the desired humanized antibody from the culture supernatant.

### <Human Antibody>

In another preferred mode of the anti-RGMa neutralizing antibody of the present invention, there is a human antibody. The term "human antibody" refers to an antibody in which the entire region including heavy chain variable regions and heavy chain constant regions and light chain variable regions and light chain constant regions constituting the immunoglobulin are derived from genes encoding human immunoglobulins. Human antibodies can be prepared by introducing human antibody genes into mice. Human antibodies can be produced in the same manner as the above-described method for preparing polyclonal antibodies or monoclonal antibodies. Specifically, for example, the method comprises introducing at least human immunoglobulin genes into gene loci of a non-human mammal such as a mouse to prepare a transgenic animal and immunizing the transgenic animal with an antigen.

For example, a transgenic mouse that produces a human antibody can be prepared according to the methods described, for example, in Nature Genetics, Vol. 7, p. 13-21, 1994; Nature Genetics, Vol. 15, p. 146-156, 1997; Japanese Translated PCT Patent Application Laid-open Nos. 1992-504365 and 1995-509137; WO94/25585; Nature, Vol. 368, p. 856-859, 1994; and Japanese Translated PCT Patent Application Laid-open No. 1994-500233. More specifically, for example, HuMab (Registered Trademark) Mouse (Medarex, Princeton, NJ), KMTM mouse (Kirin Pharma Company, Japan), and KM (FCγRIIb-KO) mouse may be used.

Specific examples of the anti-RGMa neutralizing antibody of the present invention include those having CDRs comprising specific amino acid sequences in the heavy chain variable region, and CDRs comprising specific amino acid sequences in the light chain variable region (preferably the anti-RGMa neutralizing antibodies (a) to (1) described above and (a') to (i') described above).

The amino acid sequence of the anti-RGMa neutralizing antibody (preferably the anti-RGMa neutralizing antibodies (a) to (1) described above and (a') to (i') described above) may have substitution, deletion, addition or insertion of one or several (1 to 20, 1 to 10, or 1 to 5, but preferably 1 or 2) amino acids as long as the antibody of the present invention maintains the characteristics of having an ability to bind to RGMa and inhibiting (neutralizing) the activity of RGMa. The substitution, deletion, or addition may be introduced in CDRs. However, it is preferably introduced in a region other than CDR. The amino acid substitution is preferably conservative substitution in order to maintain the characteristics of the present invention.

When the amino acid sequence of the anti-RGMa neutralizing antibody of the present invention (preferably the anti-RGMa neutralizing antibodies (a) to (1) described above and (a') to (i') described above) has a substitution, deletion, or the like therein, the amino acid sequence of the heavy chain variable region after the modification of the amino acid sequence has a % identity of 90% or more (more preferably 95%, 96%, 97%, 98%, or 99% or more) to the amino acid sequence before the modification, and the amino acid sequence of the light chain variable region after the modification of the amino acid sequence has a % identity of 90% or more (more preferably 95%, 96%, 97%, 98%, or 99% or more) to the amino acid sequence before the modification.

In the present invention the term "siRNA" refers to a short double strand RNA capable of inhibiting the expression of a target gene (an RGMa gene in the present invention). The nucleotide sequence and the length (base length) are not particularly limited as long as the function as an siRNA to inhibit the RGMa activity in the present invention is maintained. The base length is preferably less than about 30 bases, more preferably about 19 to 27 bases, and still more preferably about 21 to 25 bases.

In the present invention the term "shRNA" refers to a molecule with about 20 or more base pairs, which is a single strand RNA comprising a part having a palindromic nucleotide sequence to form a double-stranded structure in the molecule, and has a short hairpin structure with a 3'-terminal overhang. Such an shRNA can be introduced into a cell and then degraded into a length of about 20 bases (typically, for example, 21, 22, or 23 bases) in the cell to inhibit the expression of a target gene similarly to siRNA.

The siRNA and shRNA in the present invention may be in any form as long as they can inhibit the expression of the RGMa gene.

In the present invention, a siRNA or shRNA can be chemically synthesized by an artificial means. Antisense and sense RNAs can also be synthesized *in vitro* from DNA templates using, for example, a T7 RNA polymerase and a T7 promoter. The antisense oligonucleotide may be any nucleotide that is complementary to, or hybridizes to a consecutive nucleotide sequence having a length from 5 to 100 in the DNA sequence of an RGMa gene, and may be DNA or RNA. The antisense oligonucleotide may be modified insofar as its function is not impaired. The antisense oligonucleotide can be synthesized by a conventional method, and for example, it can be easily synthesized with a commercially available DNA synthesizer.

A preferred sequence can be selected by a common selection method, and the validation as the siRNA or shRNA according to the present invention can be made by evaluating inhibition of the expression of a functional RGMa.

### <Agent For Inhibiting Abnormal Protein Aggregate Accumulation>

In an embodiment of the present invention, an agent for inhibiting abnormal protein aggregate accumulation means a drug or pharmaceutical composition having the effect of inhibiting aggregates from accumulating in a cell such as a neuron, in which the aggregates are formed of abnormal proteins (misfolded proteins and mutated proteins such as TDP-43, Tau, α-synuclein, and SOD1). A neurodegenerative disease to be treated by the abnormal protein aggregate accumulation inhibiting effect according to the present invention is a disease associated with accumulation of abnormal protein aggregates. Such a disease specifically means a disease such as spinocerebellar degeneration, spinocerebellar ataxia, dentatorubropallidoluysian atrophy, fragile X-associated tremor/ataxia syndrome, spinobulbar muscular atrophy, fragile X syndrome, fragile XE syndrome, myotonic dystrophy (type 1), lethal insomnia, frontotemporal dementia or frontotemporal lobar degeneration, Pick disease, primary progressive aphasia, primary progressive nonfluent aphasia, progressive supranuclear palsy, corticobasal degeneration, argyrophilic grain dementia, globular glial tauopathy, or dementia with Lewy body. Among these, frontotemporal dementia (FTD) or frontotemporal lobar degeneration (FTLD) is a preferable example.

### <Agent for Inhibiting Uptake of Abnormal Protein into Neuron>

In an embodiment of the present invention, an agent for inhibiting the uptake of an abnormal protein into a neuron means a drug or pharmaceutical composition having the effect of inhibiting the uptake of an abnormal protein (having the same meaning as above) present outside a neuron into a neuron. Here, abnormal proteins taken into a neuron accumulate in the form of an aggregate in the cell, and thereby developing a neurodegenerative disease. Accordingly, inhibiting the uptake of an abnormal protein makes it possible to inhibit the accumulation of abnormal protein aggregates in a neuron. In the present invention, examples of neurodegenerative diseases to be treated by the effect of inhibiting the uptake of an abnormal protein into a neuron include the above-described diseases associated with accumulation of abnormal protein aggregates. Among these, frontotemporal dementia (FTD) or frontotemporal lobar degeneration (FTLD) is a preferable example.

In addition, as illustrated in the below-described Examples, an RGMa induces the dephosphorylation of cofilin, and facilitates the depolymerization from fibrous actin (F-actin) to globular actin (G-actin, also referred to as monomeric actin), thus increasing the uptake of an abnormal protein (Tau or the like) from outside a neuron into the cell. In the present invention, an RGMa inhibiting substance, particularly an anti-RGMa neutralizing antibody has the effect of inhibiting the depolymerization from fibrous actin to globular actin. In addition, an RGMa inhibiting substance in the present invention, particularly an anti-RGMa neutralizing antibody has the effect of inhibiting the uptake of an abnormal protein, which is increased by an RGMa, into a neuron by changing such actin dynamics. By virtue of these effects, an RGMa inhibiting substance in the present invention, particularly an anti-RGMa neutralizing antibody has the effect of inhibiting the accumulation of abnormal protein aggregates, and can become an agent for preventing or treating a disease associated with accumulation of abnormal protein aggregates.

As described above, the present invention encompasses an agent for inhibiting abnormal protein aggregate accumulation comprising an RGMa inhibiting substance, particularly an anti-RGMa neutralizing antibody as an active ingredient. In addition, the present invention encompasses an agent for inhibiting the uptake of an abnormal protein into a neuron, the agent comprising an RGMa inhibiting substance, particularly an anti-RGMa neutralizing antibody as an active ingredient. Furthermore, the present invention encompasses an agent for inhibiting depolymerization from fibrous actin to globular actin (preferably, an agent for inhibiting depolymerization from fibrous actin to globular actin in a neuron), the agent comprising an RGMa inhibiting substance, particularly an anti-RGMa neutralizing antibody as an active ingredient.

### <Disease in which Accumulation of Abnormal Protein Aggregates is Involved>

In an embodiment of the present invention, a disease associated with accumulation of abnormal protein aggregates has the same meaning as the above-described disease, and specifically means a disease such as spinocerebellar degeneration, spinocerebellar ataxia, dentatorubropallidoluysian atrophy, fragile X-associated tremor/ataxia syndrome, spinobulbar muscular atrophy, fragile X syndrome, fragile XE syndrome, myotonic dystrophy (type 1), lethal insomnia, frontotemporal dementia or frontotemporal lobar degeneration, Pick disease, primary progressive aphasia, primary progressive nonfluent-variant aphasia, progressive supranuclear palsy, corticobasal degeneration, argyrophilic grain dementia, globular glial tauopathy, or dementia with Lewy body. Among these, frontotemporal dementia (FTD) or frontotemporal lobar degeneration (FTLD) is a preferable example.

As described above, the present invention encompasses an agent for preventing or treating a disease associated with accumulation of abnormal protein aggregates, the agent comprising an RGMa inhibiting substance, particularly an anti-RGMa neutralizing antibody as an active ingredient. In addition, the present invention encompasses an agent for preventing or treating frontotemporal dementia (FTD) or frontotemporal lobar degeneration (FTLD), the agent comprising an RGMa inhibiting substance, particularly an anti-RGMa neutralizing antibody as an active ingredient.

### <Pharmaceutical composition>

In the present invention, an agent for inhibiting abnormal protein aggregate accumulation or an agent for inhibiting the uptake of an abnormal protein into a neuron is usually administered in oral or parenteral form systemically or topically.

In the present invention, an agent for inhibiting abnormal protein aggregate accumulation or an agent for inhibiting the uptake of an abnormal protein into a neuron comprises an RGMa inhibiting substance as an active ingredient, and can be suitably admixed with a pharmaceutically acceptable carrier or additive to be formed into a formulation. The thus formulated pharmaceutical composition can be administered in an oral or parenteral form. Specifically, the agent can be formed into oral agents such as tablets, coated tablets, pills, powders, granules, capsules, solutions, suspensions, and emulsions; or parenteral agents such as injections, infusions, suppositories, ointments, and patches. The content ratio of the carrier or additive may be set as appropriate according to the ranges commonly used in the pharmaceutical field. There is no particular restriction on the carrier or additive to be admixed, and examples thereof include water, physiological saline, other aqueous solvents, various carriers such as aqueous or oily bases; and various additives such as excipients, binders, pH adjusters, disintegrants, absorption promoters, lubricants, colorants, flavoring agents, and perfumes.

When the RGMa inhibiting substance is an anti-RGMa neutralizing antibody, a functionally modified antibody thereof, a conjugated antibody thereof, or an antigen-binding fragment thereof, the agent is preferably formulated into an injection or infusion with a pharmaceutically acceptable carrier, and administered via a parenteral route of administration, such as an intravenous, intramuscular, intradermal, intraperitoneal, subcutaneous, or local route.

The injection or infusion containing the anti-RGMa neutralizing antibody can be, for example, used as a solution, suspension, or emulsion. Examples of the solvent therefor include distilled water for injection, physiological saline, a glucose solution, and an isotonic solution (for example, solutions of sodium chloride, potassium chloride, glycerin, mannitol, sorbitol, boracic acid, borax, and propylene glycol).

The injections or infusions containing the anti-RGMa neutralizing antibody may further contain a stabilizer, a solubilizer, a suspending agent, an emulsifier, a soothing agent, a buffer agent, a preservative, an antiseptic, a pH adjuster, etc.

Examples of the stabilizer include albumin, globulin, gelatin, mannitol, glucose, dextran, ethylene glycol, propylene glycol, ascorbic acid, sodium bisulfite, sodium thiosulfate, sodium EDTA, sodium citrate, and dibutylhydroxytoluene.

Examples of the solubilizer include alcohols (such as ethanol), polyalcohols (such as propylene glycol and polyethylene glycol), and nonionic surfactants (such as polysorbate 80 (Registered Trademark), and HCO-50).

Examples of the suspending agent include glyceryl monostearate, aluminum monostearate, methylcellulose, carboxymethylcellulose, hydroxymethylcellulose, and sodium lauryl sulfate.

Examples of the emulsifier include gum arabic, sodium alginate, and tragacanth. Examples of the soothing agent include benzylalcohol, chlorobutanol, and sorbitol.

Examples of the buffer agent include a phosphate buffer, acetate buffer, borate buffer, carbonate buffer, citrate buffer, and Tris buffer.

Examples of the preservative include methyl para-hydroxybenzoate, ethyl para-hydroxybenzoate, propyl para-hydroxybenzoate, butyl para-hydroxybenzoate, chlorobutanol, benzyl alcohol, benzalkonium chloride, sodium dehydroacetate, sodium edetate, boric acid, and borax.

Examples of the antiseptic include benzalkonium chloride, para-hydroxybenzoic acid, and chlorobutanol.

Examples of the pH adjuster include hydrochloric acid, sodium hydroxide, phosphoric acid, and acetic acid.

When the RGMa inhibiting substance is a nucleic acid (such as siRNA, shRNA, and antisense oligonucleotide), it can be administered in the form of a nonviral or viral vector. When it is in the form of a nonviral vector, a method of introducing a nucleic acid molecule using a liposome (such as a liposome method, an HVJ-liposome method, a cationic liposome method, a lipofection method, or a lipofectamine method), a microinjection method, a method of transferring a nucleic acid molecule with a carrier (a metal particle) into a cell using a gene gun or the like, can be used. When siRNA or shRNA is administered to a living organism using a viral vector, a viral vector such as a recombinant adenovirus or retrovirus can be used. A DNA that expresses the siRNA or shRNA is introduced to a DNA virus or RNA virus such as detoxified retrovirus, adenovirus, adeno-associated virus, herpesvirus, vaccinia virus, poxvirus, poliovirus, sindbis virus, Sendai virus, or SV40. By infecting a cell or a tissue with the recombinant virus, the gene can be introduced into the cell or tissue.

The formulation thus obtained can be administered to, for example, a human or another mammal (such as a rat, mouse, rabbit, sheep, pig, cattle, cat, dog, or monkey) at an effective dose to prevent or treat a disease in which the accumulation of abnormal protein aggregates is involved. The dose is set as appropriate depending on the purpose, the severity of a disease, the age, weight, gender, and past history of a patient, the type of an active ingredient, or the like. For example, when the active ingredient is an anti-RGMa neutralizing antibody, the dose for an average human having a weight of about 65 to 70 kg is preferably about 0.02 mg to 4000 mg per day. The total dose per day may be administered in a single dose or in divided doses.

### EXAMPLES

The present invention will be described in more detail below with reference to Examples, which do not limit the scope of the present invention.

As an anti-RGMa neutralizing antibodies, an anti-RGMa neutralizing antibody including the amino acid sequence of (b) (Sequence number 11~15, and the amino acid sequence of AGS as HCDR3) described herein was used in Example 4, and an anti-RGMa neutralizing antibody including the amino acid sequence of (a) (SEQ ID NO: 5-10) was used in the other Examples. Palivizumab was used as a control antibody in each Example.

### Example 1: Immunohistochemistry on Paraffin-Embedded Section of mSOD1 (Mutated SOD1) Mouse Spinal Cord

To female SOD1 G93A mutant transgenic mice (mSOD1 mice), 250 µg per mouse of an anti-RGMa neutralizing antibody or control antibody was intraperitoneally administered twice a week from the time when the mice were 6 weeks old to the time when the mice manifested complete body paralysis. The mSOD 1 mice were euthanized at the age of 105 days, and then perfused with PBS containing 4% paraformaldehyde (PFA). The spinal cords were dissected, dehydrated, and then embedded in paraffin. Using a microtome, the spinal cord was made into sections with a thickness of 3 µm.

The sections were deparaffinized, washed with phosphate buffered saline (PBS), and then stained in Nissl staining with cresylviolet. Using 15 sections per individual, the number of motor neurons present in the ventral horn of the spinal cord was measured under a microscope. The results are shown in FIG. 1.

In the immunostaining, the deparaffinized sections were incubated with a 0.3% hydrogen peroxide solution for 30 minutes to quench the endogenous peroxidase activity, and then washed with phosphate buffered saline (PBS). In the immunohistochemistry, the primary antibody was used rabbit antibody against ubiquitin (DAKO), and goat anti-rabbit immunoglobulins conjugated to peroxidase-labeled dextran polymer (Dako Envision+, Dako) was used as the secondary antibody. The sections underwent 3,3'-diaminobenzidine (DAB) coloring reaction, and then were dehydrated, permeabilized, and mounted. In addition, microscopic images were taken, and then Image-J was used to quantitate the ratio of the area exhibiting a ubiquitin immunoreactivity in the ventral horn of the spinal cord. The anti-RGMa neutralizing antibody-treated mSOD1 mice (n=7) and the control antibody-treated mSOD1 mice (n=3) were compared with one another. The results are shown in FIG. 2.

In the mSOD 1 mice treated with anti-RGMa neutralizing antibody group (n = 5), the number of motor neurons in the ventral horn increased compared with the mSOD 1 mice treated with control antibody group (n = 5), and in the ventral horn of the mSOD1 mice treated with control antibody group, atrophied motor neurons were observed (FIG. 1). In the mSOD 1 mice treated with anti-RGMa neutralizing antibody group, ubiquitinated aggregate proteins were significantly decreased compared with control antibody -treated group (FIG. 2). This result has shown that anti-RGMa neutralizing antibody therapy exerts a motor neuroprotective effect on motor neuron by inhibiting abnormal protein aggregation in mSOD1 mouse model. Also in FTD, ubiquitinated aggregate proteins emerged, thus demonstrating that an anti-RGMa neutralizing antibody achieves the effect in the same manner as with the mSOD1 mice.

### Example 2: Effect on Actin Dynamics

RGMa signals regulate small GTPases, resulting in the alteration of actin dynamics. In addition, cofilin induces the depolymerization of actin, but when phosphorylated, cofilin loses such an effect.

The phosphorylation status of cofilin in the spinal cord of mSOD1 mice was examined. The mSOD1 mice treated with anti-RGMa neutralizing antibody or with control antibody (n = 5 for each group) and wild type mice (WT, n = 4, 105 days old) were perfused with PBS at 4°C. The whole spinal cord was dissected, then homogenized on ice, and stored at -80°C. These samples were lysed with RIPA buffer solution (PBS, 0.1% Triton X-100, 0.5% sodium deoxycholate, 0.1% sodium dodecyl sulfate (SDS), 50 mM Tris-HCl, and 150 mM NaCl, pH 8.0) containing protease phosphatase inhibitors. Equal concentrations of proteins were resolvedon 10% SDS-polyacrylamide gel, and transferred onto polyvinylidene fluoride (PVDF) membranes. The blots were incubated at 4°C overnight with one of the following primary antibodies: anti-cofilin antibody (1: 80000, Proteintech; 66057-1-Ig), anti-phosphorylated cofilin antibody (1: 1000, Cell Signaling; #3313). The blots were subsequently incubated with a peroxidase labeled secondary antibodies for 90 minutes, and visualized using Super Signal West Femto Maximum Sensitivity Substrate (Thermo Fisher Scientific Inc.). The image of each band was captured using Image Gauge (Fuji Film), and analyzed using ImageJ. The results are shown in FIG. 3(A) and FIG. 3(B).

Next, the state of the actin polymerization in the spinal cord was examined. Frozen sections of the spinal cords of the mSOD1 mice treated with anti-RGMa neutralizing antibody (n = 4) or control antibody (n = 4), and WT (n = 3) were fixed with 4% PFA, and exposed to 0.5% triton for 15 minutes. After three wash with PBS, phalloidin conjugated with rhodamine (Cytoskelton, #PHDR1) (1:100) was applied at 4°C overnight. The samples were mounted with Vectashield Mounting Medium containing DAPI (Vector Laboratories, H-1200). The number of phalloidin-positive cells in both the ventral horns was counted using an all-in-one microscope (BZ-X810, Keyence) with a 40× magnification. The results are shown in FIG. 3(C).

An examination was also made using the primary culture of rat neurons. The culture method is as follows.

From the 16th-day embryo ofWistar rats, cultures of primary cortical neuron were prepared. These cells were seeded in a chamber slide coated with poly-L-lysine (Sigma, P1274-25MG), and cultured in a high-glucose DMEM containing 5% fetal bovine serum and 100 IU/ml penicillin at 37°C under 5% CO₂. The culture medium was changed to the differentiation medium (Neuro Stem Cell Basal Medium (Merck, SCM003)) supplemented with B-27 (Invitrogen, 12587010), and half of themedium change was performed every two to three days. The cells were cultured for seven days, and then used for evaluation.

The influence of RGMa on the filamentary actin (F-actin) and globular actin (G-actin) in neurons was evaluated. Cells were treated recombinant human RGMa (2 µg/ml) (NovoPro, 500040) or DDW (double distilled water) and anti-RGMa neutralizing antibody or control antibody (20 µg/ml) for 30 minutes, and then were fixed with methanol and acetone at -20°C for 20 minutes. F-actin was labeled with phalloidin conjugated with rhodamine (PHDR1) for 40 minutes, and permeation treatment was perfomed with 0.1% TritonX-100 for 5 minutes, and then, the G-actin was stained with Dnase1 conjugated with Alexa Fluor (trademark) 488 (D12371) for 20 minutes . Images were obtained using a confocal microscope ZEISS LSM 880 with Airyscan (Az science). Using ImageJ, the F-actin/G-actin ratio (F/G ratio) was measured by quantitating the fluorescence intensities of rhodamine and AlexaFluor (trademark) 488 on 36 neurons obtained from three independent wells, from the images obtained with an all-in-one fluorescence microscope (BZ-X800, Keyence). The results are shown in FIG. 4(A).

In the spinal cords of the mSOD1 mice, the dephosphorylation of cofilin increased significantly, and this was inhibited by the anti-RGMa neutralizing antibody (FIG. 3(A) and (B)). F-actin staining revealed that actin depolymerization was increased in the spinal cords of mSOD1 mice, which was cancelled by the anti-RGMa neutralizing antibody (FIG. 3(C)). In the rat primary neuron culture, the F/G ratio was decreased by the addition of the RGMa, and this effect was inhibited by the anti-RGMa neutralizing antibody (FIG. 4(A)).

These results have revealed that the signal of the RGMa promotes the depolymerization of actin via the dephosphorylation of cofilin, and in addition, that the anti-RGMa neutralizing antibody cancels these effects.

### Example 3: Effect on Cellular Uptake of Extracellular Aggregated Protein and the Like

An examination was conducted using the primary culture of rat neuron in the same method as described above.

### (1) RGMa-caused Cellular Uptake of Mutated Protein via Actin Signal

The influence of RGMa on the uptake of mutated proteins was examined using Jasplakinolide (AdipoGen Life Sciences, Inc., AG-CN2-0037) , actin depolymerization inhibitor., First, recombinant SOD1 proteins (G93A, A4V) with a FLAG tag were purified. Recombinant human RGMa (2 µg/ml) or DDW (double distilled water) and Jasplakinolide (250 nM) or DMSO were added to rat primary cortical neurons and allowed to react for 30 minutes, and then, a mutated SOD1 protein (G93A, A4V) was added at 250 ng/ml. After one hour, they were fixed with methanol/acetone, and labeled with a mouse monoclonal anti-FLAG M2 antibody conjugated with FITC (Sigma-Aldrich, F4049). From images obtained using a confocal laser scanning microscope FV3000 of an inverted type (Olympus Corporation), the average fluorescence intensities of FITC in 36 neurons from three independent wells were determined using ImageJ. The results are shown in FIG. 4(B).

### (2) Effect of Anti-RGMa Neutralizing Antibody on Cellular Uptake of Mutated Protein and the Like

Whether the RGMa-caused cellular uptake of mutated proteinwas inhibited by anti-RGMa neutralizing antibody was examined. Rat primary cortical neurons were incubated with a mixture obtained by mixing recombinant FLAG labeled protein (250 ng/ml) of G93A mutated SOD1, A4V mutated SOD1, or wild type SOD1 with recombinant human RGMa (2 µg/ml), and anti-RGMa neutralizing antibody or control antibody (20 µg/ml) for 60 minutes, and fixed with methanol and acetone at -20°C for 20 minutes. The neurons were labeled with a mouse monoclonal anti-FLAG M2 antibody conjugated with FITC (Sigma-Aldrich, F4049). From images obtained using a confocal laser scanning microscope FV3000 of an inverted type (Olympus Corporation), the average fluorescence intensities of the FITC in 36 neurons from three independent wells were determined using ImageJ. The results are shown in FIG. 5. The same study was performed using dextran (pHrodo (trademark) Red Dextran, Invitrogen, P10361) instead of mutated proteins. The cells were reacted with dextran for 10 minutes. The results are shown in FIG. 6.

In the case of Tau, the rat primary cortical neurons were treated with recombinant human RGMa (2 µg/ml) and anti-RGMa neutralizing antibody or control antibody (20 µg/ml), and then, a recombinant human Tau protein (AnaSpec, AS-55556-50) was added. After 60 minutes of incubation, they were fixed with methanol and acetone at -20°C for 20 minutes. The cells were labeled with a mouse monoclonal anti-phosphorylated Tau antibody (Invitrogen MN1020 1: 100) at 4°C overnight, and then stained with Alexa Fluor (trademark) 594. The specimens were mounted using a mounting agent containing DAPI (Vector Laboratories, Inc., VECTASHIELD Mounting Medium H-1200), and images were obtained using a confocal laser scanning microscope FV3000 of an inverted type (Olympus Corporation). The average fluorescence intensities in 36 neurons from two independent wells were determined using ImageJ. The results are shown in FIG. 8.

Cellular uptake of the G93A mutant and A4V mutant SOD1 proteins were increased by the addition of RGMa, and this effect was inhibited by Jasplakinolide. This result suggests that RGMa promotes entry of mutated protein into cells by depolymerization of actin.

In the presence of RGMa, uptake of the G93A mutant and A4V mutant SOD1 proteins in cells treated with anti-RGMa neutralizing antibody was significantly decreased compared with cells treated with control antibody. (FIG. 5). In a case where the wild type SOD1 protein was used, such an increase in cellular uptake due to the addition ofRGMa was not observed (FIG. 4(B)). In addition, in a case where the cells were stimulated with dextran for 10 minutes, the same result as of the reaction presented by the mutated SOD1 protein (FIG. 6) was obtained. Also in a case where the recombinant human Tau protein was used, an increase caused by the addition of a recombinant human RGMa in cellular uptake of Tau protein was observed. In the presence of RGMa, cellular uptake of tau protein was significantly decreased in cells treated with anti-RGMa neutralizing antibody compared with cells treated with control antibody. (FIG. 7). These results have revealed thatRGMa promotes, entry of extracellularabnormal proteins into the neuron, and furthermore, that an anti-RGMa neutralizing antibody inhibits this process to achieve the effect.

These results have revealed that RGMa changes the actin dynamics in a neuron to thereby promote cellular uptake of extracellular abnormal proteins, and furthermore, that anti-RGMa neutralizing antibody inhibits this process to achieve the effect. The results have revealed that, also on FTD, an anti-RGMa neutralizing antibody achieves the effect in the same manner because Tau and the like propagate between the cells.

### Example 4: Effect of anti-RGMa neutralizing antibody on TDP-43 aggregation in mPFN1 (mutant PFN1) mice

G118V mutant profilinl-transgenic mice (mPFN1 mice) were used as a mouse model of TDP-43 aggregation. mPFN1 mice are known to have abnormal aggregation of TDP-43 in neurons. mPFN1 mice were treated with anti-RGMa neutralizing antibody or negative control antibody at 10 mg/kg dose intraperitoneally twice a week. At the end of life stage, mPFN1 mice were euthanized, perfused with 4% paraformaldehyde (PFA)-containing PBS, and the spinal cord was dissected and paraffin-embedded after dehydration. Sections of the spinal cord were prepared at a thickness of 3 µm using a microtome.

Since TDP-43 accumulates in the cytoplasm with phosphorylation, TDP-43 aggregation in neurons was detected by immunohistochemical staining for phosphorylated TDP-43. Deparaffinized sections were microwaved in 10 mM citrate buffer for antigen activation. They were then incubated with 0.3% hydrogen peroxide solution for 30 minutes to quench endogenous peroxidase activity and washed with phosphate buffered saline (PBS). A rabbit antibody against phosphorylated TDP-43 (Proteintech) was used as a primary antibody and a goat anti-rabbit immunoglobulin conjugated to peroxidase-conjugated dextran polymer (Dako Envision+, Dako) was used as a secondary antibody. 3,3'-diaminobenzidine (DAB ) was used for coloration reaction, followed by dehydration, permeabilization, and mounting. After microscopic images were taken, the percentage of spinal cord ventral horn cells positive for phosphorylated TDP-43 in their cytoplasm was visually measured by a blinded evaluator and compared between the m PFN1 mice treated with anti-RGMa neutralizing antibody (2 mice) and those treated with control antibody (3 mice). The results are shown in Fig. 8.

. The number of cytoplasmic phosphorylated TDP-43 positive cells was significantly decreased in the ventral horn of the spinal cord of m PFN1 mice treated with anti-RGMa neutralizing antibody compared to the group treated with control antibody (Figure 8). These results demonstrate that anti-RGMa neutralizing antibody therapy inhibits TDP-43 aggregation, and suggest that anti-RGMa neutralizing antibodies are also effective for FTD where aggregation of TDP-43 aggregating proteins is observed, as in m PFN1 mice.

### Example 5: Evaluation Made Using Clinical Sample (Measurement of RGMa in CSF)

From nine FTD patients and control patients (32 patients withother neurodegenerative disorders (OND) and 24 patients withnon-neurodegenerative disorders (ND)), the cerebrospinal fluid was collected, and 400 g of the cerebrospinal fluid was immediately centrifuged at 4°C for 10 minutes. The supernatant of CSF was preserved at -80°C until use. The patients of other neurodegenerative disorders (OND) were patients with multiple sclerosis, neuromyelitis optica, Parkinson's disease, chronic inflammatory demyelinating polyradiculoneuropathy, or cervical spondylosis, and the patients of non-neurodegenerative disorders (ND) were patients with normal pressure hydrocephalus, myopathy, peripheral nerve disease, or dissociative disorder.

The concentration of a soluble type RGMa in the CSF was measured, using enzyme-linked immunosorbent assay (ELISA) kit (R&D Systems). The results are shown in FIG. 9.

The soluble type RGMa in the cerebrospinal fluid of the FTD patients increased significantly compared with those in patients with non-neurodegenerative disorders (ND) and other neurodegenerative disorders (OND). These data show that RGMa increased in the cerebrospinal fluid of FTD patients, and contributed to the pathology of FTD.

### <Sequence Listing>

SEQ ID NO: 1: Amino acid sequence of human RGMa precursor protein
SEQ ID NO: 2: Amino acid sequence of murine RGMa precursor protein
SEQ ID NO: 3: Amino acid sequence of rat RGMa precursor protein
SEQ ID NO: 4: DNA sequence of human RGMa gene
SEQ ID NO: 5: Amino acid sequence of LCDR1 of anti-RGMa neutralizing antibody r116A3
SEQ ID NO: 6: Amino acid sequence of LCDR2 of anti-RGMa neutralizing antibody r116A3
SEQ ID NO: 7: Amino acid sequence of LCDR3 of anti-RGMa neutralizing antibody r116A3
SEQ ID NO: 8: Amino acid sequence of HCDR1 of anti-RGMa neutralizing antibody r116A3
SEQ ID NO: 9: Amino acid sequence of HCDR2 of anti-RGMa neutralizing antibody r116A3
SEQ ID NO: 10: Amino acid sequence of HCDR3 of anti-RGMa neutralizing antibody r116A3
SEQ ID NO: 11: Amino acid sequence of LCDR1 of anti-RGMa neutralizing antibody r70E4
SEQ ID NO: 12: Amino acid sequence of LCDR2 of anti-RGMa neutralizing antibody r70E4
SEQ ID NO: 13: Amino acid sequence of LCDR3 of anti-RGMa neutralizing antibody r70E4
SEQ ID NO: 14: Amino acid sequence of HCDR1 of anti-RGMa neutralizing antibody r70E4
SEQ ID NO: 15: Amino acid sequence of HCDR2 of anti-RGMa neutralizing antibody r70E4
SEQ ID NO: 16: Amino acid sequence of human RGMa epitope
SEQ ID NO: 17: Amino acid sequence of LCDR1 of anti-RGMa neutralizing antibody 5F9
SEQ ID NO: 18: Amino acid sequence of LCDR2 of anti-RGMa neutralizing antibody 5F9
SEQ ID NO: 19: Amino acid sequence of LCDR3 of anti-RGMa neutralizing antibody 5F9
SEQ ID NO: 20: Amino acid sequence of HCDR1 of anti-RGMa neutralizing antibody 5F9
SEQ ID NO: 21: Amino acid sequence of HCDR2 of anti-RGMa neutralizing antibody 5F9
SEQ ID NO: 22: Amino acid sequence of HCDR3 of anti-RGMa neutralizing antibody 5F9
SEQ ID NO: 23: Amino acid sequence of LCDR1 of anti-RGMa neutralizing antibody 8D1
SEQ ID NO: 24: Amino acid sequence of LCDR2 of anti-RGMa neutralizing antibody 8D1
SEQ ID NO: 25: Amino acid sequence of LCDR3 of anti-RGMa neutralizing antibody 8D1
SEQ ID NO: 26: Amino acid sequence of HCDR1 of anti-RGMa neutralizing antibody 8D1
SEQ ID NO: 27: Amino acid sequence of HCDR2 of anti-RGMa neutralizing antibody 8D1
SEQ ID NO: 28: Amino acid sequence of HCDR3 of anti-RGMa neutralizing antibody 8D1
SEQ ID NO: 29: Amino acid sequence of LCDR1 of anti-RGMa neutralizing antibody AE12-1
SEQ ID NO: 30: Amino acid sequence of LCDR2 of anti-RGMa neutralizing antibody AE12-1
SEQ ID NO: 31: Amino acid sequence of LCDR3 of anti-RGMa neutralizing antibody AE12-1
SEQ ID NO: 32: Amino acid sequence of HCDR1 of anti-RGMa neutralizing antibody AE12-1
SEQ ID NO: 33: Amino acid sequence of HCDR2 of anti-RGMa neutralizing antibody AE12-1
SEQ ID NO: 34: Amino acid sequence of HCDR3 of anti-RGMa neutralizing antibody AE12-1
SEQ ID NO: 35: Amino acid sequence of LCDR3 of anti-RGMa neutralizing antibody AE12-1Y
SEQ ID NO: 36: Amino acid sequence of human RGMa epitope
SEQ ID NO: 37: Amino acid sequence of human RGMa epitope
SEQ ID NO: 38: Amino acid sequence of human RGMa epitope
SEQ ID NO: 39: Amino acid sequence of human RGMa epitope
SEQ ID NO: 40: Amino acid sequence of LCDR3 of anti-RGMa neutralizing antibody AE12-1F
SEQ ID NO: 41: Amino acid sequence of LCDR3 of anti-RGMa neutralizing antibody AE12-1H
SEQ ID NO: 42: Amino acid sequence of LCDR3 of anti-RGMa neutralizing antibody AE12-1L
SEQ ID NO: 43: Amino acid sequence of LCDR3 of anti-RGMa neutralizing antibody AE12-1V
SEQ ID NO: 44: Amino acid sequence of LCDR3 of anti-RGMa neutralizing antibody AE12-1I
SEQ ID NO: 45: Amino acid sequence of LCDR3 of anti-RGMa neutralizing antibody AE12-1K
SEQ ID NO: 46: Amino acid sequence of light chain variable region of anti-RGMa neutralizing antibody r116A3 (humanized antibody)
SEQ ID NO: 47: Amino acid sequence of heavy chain variable region of anti-RGMa neutralizing antibody r116A3 (humanized antibody)
SEQ ID NO: 48: Amino acid sequence of light chain of anti-RGMa neutralizing antibody r116A3 (humanized antibody)
SEQ ID NO: 49: Amino acid sequence of heavy chain of anti-RGMa neutralizing antibody r116A3 (humanized antibody)
SEQ ID NO: 50: Amino acid sequence of light chain variable region of anti-RGMa neutralizing antibody r70E4
SEQ ID NO: 51: Amino acid sequence of heavy chain variable region of anti-RGMa neutralizing antibody r70E4
SEQ ID NO: 52: Amino acid sequence of light chain of anti-RGMa neutralizing antibody r70E4
SEQ ID NO: 53: Amino acid sequence of heavy chain of anti-RGMa neutralizing antibody r70E4
SEQ ID NO: 54: Amino acid sequence of light chain variable region of anti-RGMa neutralizing antibody 5F9
SEQ ID NO: 55: Amino acid sequence of heavy chain variable region of anti-RGMa neutralizing antibody 5F9
SEQ ID NO: 56: Amino acid sequence of light chain variable region of anti-RGMa neutralizing antibody 8D1
SEQ ID NO: 57: Amino acid sequence of heavy chain variable region of anti-RGMa neutralizing antibody 8D1
SEQ ID NO: 58: Amino acid sequence of light chain variable region of anti-RGMa neutralizing antibody AE12-1
SEQ ID NO: 59: Amino acid sequence of heavy chain variable region of anti-RGMa neutralizing antibody AE12-1
SEQ ID NO: 60: Amino acid sequence of light chain variable region of anti-RGMa neutralizing antibody AE12-1Y
SEQ ID NO: 61: Amino acid sequence of light chain of anti-RGMa neutralizing antibody AE12-1Y-QL
SEQ ID NO: 62: Amino acid sequence of heavy chain of anti-RGMa neutralizing antibody AE12-1Y-QL

### INDUSTRIAL APPLICABILITY

In the present invention, an RGMa inhibiting substance has the effect of inhibiting the accumulation of abnormal protein aggregates or the effect of inhibiting the uptake of abnormal proteins in a neuron. The present invention is expected to be useful for preventing or treating a disease associated with accumulation of abnormal protein aggregates. The present invention is of high utility value in the pharmaceutical industry.

## Claims

1. An agent for inhibiting abnormal protein aggregate accumulation, comprising an RGMa inhibiting substance as an active ingredient.

2. An agent for inhibiting the uptake of an abnormal protein into a neuron, comprising an RGMa inhibiting substance as an active ingredient.

3. The agent according to claim 1 or 2, wherein the agent for inhibiting abnormal protein aggregate accumulation or the agent for inhibiting the uptake of an abnormal protein into a neuron is an agent for preventing or treating a disease associated with accumulation of abnormal protein aggregates.

4. The agent according to claim 3, wherein the disease associated with accumulation of abnormal protein aggregates is a disease selected from spinocerebellar degeneration, spinocerebellar ataxia, dentatorubropallidoluysian atrophy, fragile X-associated tremor/ataxia syndrome, spinobulbar muscular atrophy, fragile X syndrome, fragile XE syndrome, myotonic dystrophy (type 1), lethal insomnia, frontotemporal dementia or frontotemporal lobar degeneration, Pick disease, primary progressive aphasia, primary progressive nonfluent-variant aphasia, progressive supranuclear palsy, corticobasal degeneration, argyrophilic grain dementia, globular glial tauopathy, and dementia with Lewy body.

5. The agent according to claim 3 or 4, wherein the disease associated with accumulation of abnormal protein aggregates is frontotemporal dementia (FTD) or frontotemporal lobar degeneration (FTLD).

6. The agent according to any one of claims 1 to 5, wherein the RGMa inhibiting substance is an anti-RGMa neutralizing antibody.

7. The agent according to claim 6, wherein the anti-RGMa neutralizing antibody is a humanized antibody.

8. The agent according to claim 6 or 7, wherein the anti-RGMa neutralizing antibody is an antibody recognizing an amino acid sequence selected from SEQ ID NOS: 16, 36, 37, 38, and 39.

9. The agent according to any one of claims 6 to 8, wherein the anti-RGMa neutralizing antibody is an antibody selected from the following (a) to (l):
(a) an anti-RGMa neutralizing antibody comprising a light chain variable region comprising an LCDR1 comprising the amino acid sequence represented by SEQ ID NO: 5, an LCDR2 comprising the amino acid sequence represented by SEQ ID NO: 6, and an LCDR3 comprising the amino acid sequence represented by SEQ ID NO: 7, and a heavy chain variable region comprising an HCDR1 comprising the amino acid sequence represented by SEQ ID NO: 8, an HCDR2 comprising the amino acid sequence represented by SEQ ID NO: 9, and an HCDR3 comprising the amino acid sequence represented by SEQ ID NO: 10;
(b) an anti-RGMa neutralizing antibody comprising a light chain variable region comprising an LCDR1 comprising the amino acid sequence represented by SEQ ID NO: 11, an LCDR2 comprising the amino acid sequence represented by SEQ ID NO: 12, and an LCDR3 comprising the amino acid sequence represented by SEQ ID NO: 13, and a heavy chain variable region comprising an HCDR1 comprising the amino acid sequence represented by SEQ ID NO: 14, an HCDR2 comprising the amino acid sequence represented by SEQ ID NO: 15, and an HCDR3 comprising an amino acid sequence comprising SFG;
(c) an anti-RGMa neutralizing antibody comprising a light chain variable region comprising an LCDR1 comprising the amino acid sequence represented by SEQ ID NO: 17, an LCDR2 comprising the amino acid sequence represented by SEQ ID NO: 18, and an LCDR3 comprising the amino acid sequence represented by SEQ ID NO: 19, and a heavy chain variable region comprising an HCDR1 comprising the amino acid sequence represented by SEQ ID NO: 20, an HCDR2 comprising the amino acid sequence represented by SEQ ID NO: 21, and an HCDR3 comprising the amino acid sequence represented by SEQ ID NO: 22;
(d) an anti-RGMa neutralizing antibody comprising a light chain variable region comprising an LCDR1 comprising the amino acid sequence represented by SEQ ID NO: 23, an LCDR2 comprising the amino acid sequence represented by SEQ ID NO: 24, and an LCDR3 comprising the amino acid sequence represented by SEQ ID NO: 25, and a heavy chain variable region comprising an HCDR1 comprising the amino acid sequence represented by SEQ ID NO: 26, an HCDR2 comprising the amino acid sequence represented by SEQ ID NO: 27, and an HCDR3 comprising the amino acid sequence represented by SEQ ID NO: 28;
(e) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 31, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(f) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 35, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(g) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 40, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(h) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 41, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(i) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 42, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(j) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 43, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34;
(k) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30, and an LCDR3 comprising the amino acid sequence represented by SEQ ID NO: 44, and a heavy chain variable region comprising an HCDR1 comprising the amino acid sequence represented by SEQ ID NO: 32, an HCDR2 comprising the amino acid sequence represented by SEQ ID NO: 33, and an HCDR3 comprising the amino acid sequence represented by SEQ ID NO: 34; and
(l) an anti-RGMa neutralizing antibody comprising a light chain variable region comprising an LCDR1 comprising the amino acid sequence represented by SEQ ID NO: 29, an LCDR2 comprising the amino acid sequence represented by SEQ ID NO: 30 and an LCDR3 comprising the amino acid sequence represented by SEQ ID NO: 45, and a heavy chain variable region comprising an HCDR1 comprising the amino acid sequence represented by SEQ ID NO: 32, an HCDR2 comprising the amino acid sequence represented by SEQ ID NO: 33 and an HCDR3 comprising the amino acid sequence represented by SEQ ID NO: 34.

10. A method of preventing or treating a disease associated with accumulation of abnormal protein aggregates, the method comprising administering an effective dose of an RGMa inhibiting substance to a mammal in need of treatment.

11. The preventive or therapeutic method according to claim 10, wherein the RGMa inhibiting substance is an anti-RGMa neutralizing antibody.

12. Use of an RGMa inhibiting substance in manufacture of an agent for preventing or treating a disease associated with accumulation of abnormal protein aggregates.

13. The use according to claim 12, wherein the RGMa inhibiting substance is an anti-RGMa neutralizing antibody.
